# EUROPEAN PATENT APPLICATION

(11) **EP 4 748 314 A1**
(43) Date of publication of application: **27.05.2026**
(21) Application number: 24214068.9
(22) Date of filing: 20.11.2024
(51) Int. Cl.: A61B 5/389, A61B 5/394, A61B 5/00, A61F 2/54, A61F 2/72, G06F 3/01

(54) **COMPUTER-IMPLEMENTED METHOD FOR ASSOCIATING A HUMAN BODY GESTURE TO A MYO- ELECTRIC SIGNAL, AS WELL AS ORTHOPEDIC ASSISTIVE DEVICE AND CLIENT-SERVER SYSTEM ADAPTED TO PERFORM SAID METHOD**

(71) Applicant: Universität der Bundeswehr München, 85579 Neubiberg (DE)
(72) Inventor: BORGHOFF, Uwe, 85635 Höhenkirchen (DE); BUCHENRIEDER, Klaus, 85521 Riemerling (DE)
(74) Representative: Lucke, Andreas

(57) **Abstract**

A computer-implemented method is provided for associating a human body gesture to a detected myoelectric signal. The method comprises: detecting at least a part of a myoelectric signal as the detected myoelectric signal; and performing the following first process steps, each of said first process steps using a computer system comprised in an orthopedic assistive device, said computer system comprising at least one processor, a wireless transmission system, and a wireless reception system: deriving data from the detected myoelectric signal, the derived data representing at least part of an information-content of the detected myoelectric signal, a data volume of the derived data being smaller than a data volume of the detected myoelectric signal; sending the derived data using the wireless transmission system; and checking for a response received using the wireless reception system, said response comprising a first human body gesture. The method further comprises performing, before, after, during, or in between the first process steps, using the computer system comprised in the orthopedic assistive device, a recognition process to determine a second human body gesture corresponding to the detected myoelectric signal; and determining, by the computer system, the one of the first human body gesture and the second human body gesture which is first available at the computer system as the human body gesture associated with the detected myoelectric signal.

## Description

### FIELD OF THE DISCLOSURE

The present disclosure relates to a system and a method for classification of a detected myoelectric signal, or for associating a human body gesture with the detected myoelectric signal, respectively. Specifically, the present disclosure pertains to an orthopedic assistive device equipped with a computer system for processing the myoelectric signal locally, and also sending it to a server for remote gesture recognition.

### BACKGROUND

A myoelectric signal, also referred to as MES throughout this disclosure, is an electric signal generated in an animal or human body in the course of muscle contraction. The myoelectric signal can be measured using an electromyograph. The electromyograph applies electrodes connected to the animal or human body, typically on the surface of the skin, to measure said electric signals, typically as a voltage difference between at least one pair of said electrodes. Any gesture carried out by the human/animal body gives rise to a corresponding MES.

Identification of the gesture based on the MES holds the potential to control or steer various orthopedic assistive devices, such as an orthosis, an exoskeleton or a prosthesis, based solely or at least in part on the MES measured by the electromyograph via the electrodes. In some situations, an additional controller such as a keyboard, a steering wheel, or a microphone can be avoided. In other words, a requirement to steer said devices by manual or voice control may be avoided, using only the electromyograph with the electrodes.

Although significant progress has been made in this field, some challenges remain regarding the signal processing and analysis, and the identification of gestures from the MES. The challenges are, at least in part, a consequence of the small amplitude of the MES (typically between 50 µV and up to a few millivolt) and of the abundance of interfering signals, resulting, for example, from electrocardiographic signals related to the activity of the heart muscle, or to movements of other parts of the human/animal body inducing voltages at the electrodes. These signal artifacts complicate the identification of gestures from the MES significantly.

### SUMMARY OF THE DISCLOSURE

In view of the technical problems laid out above, there is a need for improvements in relating an MES to a gesture of a human body. This objective is achieved with the method of claim 1, the orthopedic assistive device of claim 11, the client-server system of claim 14, and the computer program of claim 15. The dependent claims lay out preferred embodiments.

A first aspect of the invention provides a computer-implemented method for associating a human body gesture to a detected myoelectric signal. The method comprises: detecting at least a part of a myoelectric signal as the detected myoelectric signal; and performing the following first process steps, each of said first process steps using a computer system comprised in an orthopedic assistive device, said computer system comprising at least one processor, a wireless transmission system, and a wireless reception system: deriving data from the detected myoelectric signal, the derived data representing at least part of an information-content of the detected myoelectric signal, a data volume of the derived data being smaller than a data volume of the detected myoelectric signal; sending the derived data using the wireless transmission system; and checking for a response received using the wireless reception system, said response comprising a first human body gesture. The method further comprises performing, before, after, during, or in between the first process steps, using the computer system comprised in the orthopedic assistive device, a recognition process to determine a second human body gesture corresponding to the detected myoelectric signal; and determining, by the computer system, the one of the first human body gesture and the second human body gesture which is first available at the computer system as the human body gesture associated with the detected myoelectric signal.

The recognition process to determine the second human body gesture corresponding to the detected myoelectric signal may refer to a classification of the detected myoelectric signal, in particular with respect to classes corresponding to human body gestures, and assigning the result of the classification to the detected myoelectric signal as the second human body gesture. In other words, the recognition process to determine the second human body gesture corresponding to the detected myoelectric signal may refer to performing a classification on the second human body gesture, and associating the second human body gesture to the detected myoelectric signal based on the classification, in particular according to the result of the classification.

The data volume may refer to or may be expressed as a storage space required to store the respective data volume.

As laid out in detail above, a recognition process to determine a human body gesture corresponding to a detected myoelectric signal, such as the recognition process of the method according to the first aspect to determine the (first or second) human body gesture, poses a challenging classification problem. To improve the accuracy of a respective classification, or of said recognition process to determine the (first or second) human body gesture, respectively, the process is preferably carried out on a computer system having a high computing power, for example in terms of processor speed, e.g. in terms of clock cycles, number of cores for parallel processing, memory bandwidth, or storage speed. Performing the recognition process, or classification, respectively, on such a computer system having a high computing power, allows for applying a more complex and sophisticated recognition process, such as a complex and sophisticated deterministic classification scheme, and/or a larger machine learning model, generally improving the accuracy of the result of the recognition process. In particular, the use of the computer system having a high computing power allows providing a result within a time interval acceptable for satisfactory performance of the orthopedic assistive device, when applying said complex and sophisticated recognition process.

However, a computer system having a high computing power is usually not available in an orthopedic assistive device. This is because the orthopedic assistive device preferably has a minimized total weight, to increase the comfort for a user. During use of the orthopedic assistive device, such as an orthosis or a prosthesis, the user typically carries and mechanically supports the orthopedic assistive device permanently or at least for significant time intervals. For this reason, the orthopedic assistive device is preferably equipped with a computer system having a minimized weight, and, in particular, with a battery (being the power supply of said computer system) of the computer system having a minimized weight. Due to those weight limitations to the computer system in general, and in particular because of the lightweight battery, the orthopedic assistive device is preferably equipped with a computer system having a low computing power. The use of a computer system having a low computing power reduces the energy consumption of the computer system on the orthopedic assistive device, or its power consumption, respectively. This reduces the requirements to the battery of the computer system, resulting in a reduced weight of the battery and of the computer system and hence of the overall orthopedic assistive device, improving the comfort for a user.

The computer-implemented method according to the first aspect beneficially allows for the usage of a computer system of the orthopedic assistive device having a low computing power (hereinafter also referred to as the low-power computing system), and for providing a connection to a server providing a computer system having a high computing power (hereinafter also referred to as the high-power computing system) for a recognition process/classification on the detected MES of improved accuracy.

For this purpose, the data volume of the detected myoelectric signal is reduced on the computer system of the orthopedic assistive device, and the data derived this way are sent through the wireless transmission system. The low-power computing system of the orthopedic assistive device typically provides sufficient resources to reduce the data volume quickly, since the reduction of the data volume is computationally far less demanding than performing a complex and sophisticated recognition process. Because of the reduced data volume, the derived data are sent quickly. In other words, by reducing the data volume, the system can transmit information more quickly and with less bandwidth, reducing latency.

Under good reception conditions for the wireless transmission, a computer system acting as a server and having a high computing power can receive the derived data promptly, performs an additional recognition process on the derived data, and, as the respective computer system has the high computing power, the result of the additional recognition process is obtained quickly and is of good accuracy. The respective result is sent from the high-power computing system using a wireless transmission, and, under good reception conditions for the wireless transmission, is received shortly after at the computer system of the orthopedic assistive device.

In this desirable situation, a recognition/classification result of good accuracy, represented in the first human body gesture of the method according to the first aspect, is quickly available at the orthopedic assistive device for controlling the movement of the orthopedic assistive device (for example, by giving instructions to motors of the orthopedic assistive device to perform movements to mimic the recognized human body gesture).

In this context, it is worth noting that the recognition/classification in a conventional orthopedic assistive device typically takes up to two seconds, i.e., from a moment when the user performs an action that causes a corresponding signature in the MES until a human body gesture corresponding to said action has been associated with the detected MES. Under good reception conditions for the wireless transmission, the method according to the first aspect does not only improve the accuracy of the recognition/classification for the usage of a wireless transmission and reception, allowing for communication with a high-power computing system, but, under said good reception conditions, also holds the potential to provide a faster recognition/classification than the conventional techniques, making use of the potential communication with the high-power computing system.

Even in a situation in which the conventional classification/recognition of a human body gesture is relatively fast, and even if the method according to the first aspect may not obtain the first human body gesture faster than the conventional technique, the method according to the first aspect, under good reception conditions for the wireless transmission/reception, is still sufficiently fast to obtain a high-accuracy classification result (first human body gesture) from the wireless reception within a time period satisfactory for operation of the orthopedic assistive device. Notably, the operation of the motors of the orthopedic assistive device to mimic the identified human body gesture takes several seconds after the human body gesture has been associated to the myoelectric signal. Consequently, for as long as the reception conditions for the wireless transmission/reception to obtain the high-accuracy classification result (first human body gesture) within a time period of less than one second, satisfactory operation of the orthopedic assistive device is still possible.

The method beneficially allows communication with a variety of different high-power computing systems. For example, the computer system of the orthopedic assistive device may communicate with a battery-operated mobile device, such as a laptop or a smart phone, located in the proximity of the orthopedic assistive device.

Alternatively, the computer system of the orthopedic assistive device may communicate with a workstation computer system power-supply through grid power, potentially allowing for improved computing power. Respective embodiments are of particular relevance for use cases wherein the orthopedic assistive device is used in an environment where the user stays often or for extended time periods, such as at home or at a workplace.

Alternatively, the computer system of the orthopedic assistive device may communicate with a computer network such as the Internet or the World Wide Web, leveraging the resources of multiple computers of said computer network, for example through cloud computing.

In some embodiments, the high-power computing system that the computer system of the orthopedic assistive device communicates with is a multi-level system in a sense that it comprises a local computer (e.g., the battery-operated mobile device or the workstation computer system described above) and a computer network (e.g., as described above), wherein the local computer forwards at least part of the derived data to the computer network for performing at least a portion of the above-mentioned additional recognition process.

However, sending the derived data through a wireless transmission and checking for a response through a wireless reception holds the risk that this type of communication may fail, for various reasons and on various timescales. For example, the reception conditions for the wireless communication may be poor, e.g., as a battery-operated mobile device is placed unfavorably, or because a connection to the Internet through a cell phone network fails as the user and thus the orthopedic assistive device stays in a region not covered by the cell phone network. In another example, a local computer (e.g., the battery-operated mobile device or the workstation computer system described above) may fail, for example as the battery lifetime of a battery-operated mobile device is exhausted or the local computer suffers a material failure or a power outage. In any of the respective scenarios in which the wireless communication fails, the computer system of the orthopedic assistive device checking for the response received using the wireless reception system and comprising the first human body gesture will come to the conclusion that a respective first human body gesture is not available, or at least is not available within a time integral acceptable for satisfactory operation of the orthopedic assistive device.

The method according to the first aspect solves this problem by performing a recognition process using the computer system of the orthopedic assistive device to determine a second human body gesture corresponding to the detected myoelectric signal.

Preferably, the recognition process to determine the second human body gesture corresponding to the detected myoelectric signal, which is performed using the computer system comprised in the orthopedic assistive device, is performed after sending the derived data using the wireless transmission system or at least in part after sending the derived data using the wireless transmission system. In some embodiments, performing the recognition process to determine the second human body gesture corresponding to the detected myoelectric signal begins when or before the derived data are sent using the wireless transmission system.

Hence, the respective recognition process provides a fallback option to associate a human body gesture to the detected myoelectric signal, in particular, when checking, using the wireless reception system, for the response comprising the first human body gesture, fails to make the first human body gesture available.

Since the computer system of the orthopedic assistive device is generally and preferably a low-power computing system, the recognition process performed using this computer system is typically slow and/or of low accuracy. To some degree, the slowness and the accuracy of the recognition process may be balanced against each other, e.g., by using a more complex and sophisticated recognition process, which typically results in said recognition process being slower, or by using a faster recognition process, typically resulting in a reduced accuracy of the recognition process. Anyway, both a fast and a highly accurate result of the recognition process is difficult to achieve because of the computer system of the orthopedic assistive device preferably being a low-power computing system. In particular, in cases, wherein the result of the recognition process is available particularly promptly, it may be of particularly low accuracy.

The method according to the first aspect accounts for those properties of the wireless communication and of the recognition process by determining the human body gesture associated with the detected myoelectric signal as the one of the first human body gesture and the second human body gesture which is first available at the computer system. Thus, if the wireless communication is unavailable, the second human body gesture which is output from the recognition process performed on the computer system of the orthopedic assistive device is still available, keeping the orthopedic assistive device operational. If the second human body gesture should be available at the computer system of the orthopedic assistive device first, the method gives the option for further refinements, as will be described in detail below.

According to some embodiments, the method further comprises: checking, by the computer system, if a first reliability measure is available at the computer system, said first reliability measure being associated with the one of the first human body gesture and the second human body gesture which is first available at the computer system, said first reliability measure being associated with a likelihood for said one of the first human body gesture and the second human body gesture which is first available at the computer system to correspond to the detected myoelectric signal. In respective embodiments, the one of the first human body gesture and the second human body gesture which is first available at the computer system may be determined as the human body gesture associated to the detected myoelectric signal if the first reliability measure is available at the computer system and exceeds a first threshold reliability measure value.

In particular, the one of the first human body gesture and the second human body gesture which is first available at the computer system may be determined as the human body gesture associated to the detected myoelectric signal only if the first reliability measure is available at the computer system and exceeds the first threshold reliability measure value. Alternatively, or in addition, the one of the first human body gesture and the second human body gesture which is first available at the computer system may be rejected from being determined as the human body gesture associated to the detected myoelectric signal if the first reliability measure is below the first threshold reliability measure value.

Integrating a respective reliability measure into the method allows the system to evaluate the confidence level of the associated gesture. This ensures that the method does not hastily associate a gesture with a myoelectric signal without considering the accuracy of the detection. By assessing the likelihood that the detected gesture corresponds to the myoelectric signal, the method can make more informed decisions, thereby improving the overall reliability and accuracy of the classification. Requiring the first reliability measure to exceed a threshold value before associating a gesture with the detected myoelectric signal ensures that only high-confidence gestures are recognized and acted upon. This threshold mechanism acts as a filter to prevent erroneous or low-confidence gestures from being processed and executed, thereby enhancing the safety and dependability of the orthopedic assistive device.

According to an embodiment, the method further comprises, before, after, during, or in between the first process steps: calculating, using the computer system, based on the recognition process to determine the second human body gesture corresponding to the detected myoelectric signal, a second reliability measure, the second reliability measure being associated with a likelihood for the second human body gesture to correspond to the detected myoelectric signal. In respective embodiments, the second human body gesture may be determined as the human body gesture associated with the detected myoelectric signal if the second reliability measure exceeds the second threshold reliability measure value.

In particular, the second human body gesture may be determined as the human body gesture associated with the detected myoelectric signal only if the second reliability measure exceeds the second threshold reliability measure value. Alternatively, or in addition, the second human body gesture may be rejected from being determined as the human body gesture associated to the detected myoelectric signal if the second reliability measure is below the second threshold reliability measure value.

This conditional determination ensures that only highly reliable second human body gestures are used, thereby minimizing errors and enhancing the precision of the classification. This ensures that the system relies on the most accurate and reliable data available, improving safety and reliability of the method.

According to some embodiments, the recognition process to determine the second human body gesture corresponding to the detected myoelectric signal is performed using a convolutional neural network.

The inventors have performed systematic studies demonstrating that amongst various machine learning models and classification techniques assessed for performing the recognition process, a convolutional neural network outperforms the other machine learning models and classification techniques in terms of the percentage of correct assignments of the second human body gesture to the detected myoelectric signal.

In respective embodiments, the second reliability measure may be calculated using said convolutional neural network.

Beneficially, a convolutional neural network outputs a suitable second reliability measure. The use of a convolutional neural network thus allows for the reliability of the gesture recognition to be quantified, allowing the system to make more informed decisions based on the confidence level of the detected gesture, improving the robustness and accuracy of the method.

Alternatively, or in addition, the data may be derived from the detected myoelectric signal using at least a first layer of said convolutional neural network, in particular using at least one convolutional layer of said convolutional neural network and optionally further using at least one pooling layer of said convolutional neural network.

The inventors have realized that convolution and optionally pooling in a convolutional neural network provide(s) a highly efficient way to reduce the data volume of the MES, while preserving relevant features of the MES for classification/gesture recognition. Beneficially, the derived data, in the form of the convoluted and optionally pooled data, are suitable both for usage in the recognition process performed using the computer system of the orthopedic assistive device, and (because of their reduced data volume) for fast sending using the wireless transmission system. In other words, the method uses the convolution, and optionally the pooling, that it would anyway perform within the recognition process to determine the second human body gesture corresponding to the detected myoelectric signal, also for deriving the data from the detected myoelectric signal for sending those derived data using the wireless transmission system. Respective embodiments thus make twofold use of the convolution, and optionally of the pooling, in the convolutional neural network, avoiding additional process steps which would arise if the data with the reduced data volume were to be derived in a different manner.

Alternatively, or in addition, said convolutional neural network may be a convolutional neural network on the computer system of the orthopedic assistive device.

According to some embodiments, the method further comprises: checking, by the computer system, if a later one of the first human body gesture and the second human body gesture is available at the computer system; checking, by the computer system, if a third reliability measure associated with the later one of the first human body gesture and the second human body gesture is available at the computer system, the third reliability measure being associated with a likelihood for said later one of the first human body gesture and the second human body gesture to correspond to the detected myoelectric signal. In respective embodiments, the method may further comprise, if the first reliability measure, the third reliability measure and the later one of the first human body gesture and the second human body gesture are available at the computer system: if the third reliability measure exceeds the first reliability measure: changing the human body gesture associated with the detected myoelectric signal from the one of the first human body gesture and the second human body gesture which is first available at the computer system to the later one of the first human body gesture and the second human body gesture.

In respective embodiments, the human body gesture associated with the detected myoelectric signal may be changed from the one of the first human body gesture and the second human body gesture which is first available at the computer system to the later one of the first human body gesture and the second human body gesture only if a time delay between a first moment in time when the detected myoelectric signal is received at the computer system and a second moment in time when the checking if the later one of the first human body gesture and the second human body gesture is available at the computer system has found that the later one of the first human body gesture and the second human body gesture is available at the computer system does not exceed a threshold time delay, in particular, the threshold time delay may be associated with a duration of the human body gesture associated with the detected myoelectric signal, in particular, the threshold time delay corresponds to half of the duration of the human body gesture associated with the detected myoelectric signal or to 70% of the duration of the human body gesture associated with the detected myoelectric signal or to 90% of the duration of the human body gesture associated with the detected myoelectric signal or to the duration of the human body gesture associated with the detected myoelectric signal.

The later one of the first human body gesture and the second human body gesture may refer to the one of the first human body gesture and the second human body gesture that becomes available at the computer system only after the one of the first human body gesture and the second human body gesture which is first available at the computer system has become available at the computer system.

Respective embodiments ensure that the most accurate human body gesture (in terms of the reliability measures) is considered as the human body gesture associated with the detected myoelectric signal, even in cases, wherein the more accurate human body gesture (as reflected in the reliability measures) is only available later at the computer system of the orthopedic assistive device. This brings a series of advantages:

In cases where the wireless reception is only slightly delayed, for example due to a slight instability in the wireless communication, respective embodiments avoid that the recognition process performed on the orthopedic assistive device takes precedence over the first human body gesture, which may already be received shortly after the second human body gesture has become available. This is particularly relevant in embodiments wherein a fast, potentially inaccurate technique is applied in the recognition process performed using the computer system comprised in the orthopedic assistive device.

The inventors have further realized that in many cases the second human body gesture determined using the computer system of the orthopedic assistive device is similar to the first human body gesture, which may be determined using a recognition process on a high-power computing system, even if it is a different human body gesture. This is particularly true for a MES comprising multiple channels associated with multiple electrodes arranged at different positions on the human body.

The inventors hypothesize that the reason for the similarity is that a same muscle or a group of same muscles is involved in the first human body gesture and the second human body gesture, performing a similar or even identical movement, while another muscle or a group of other muscles performs dissimilar movements in the first and the second human body gesture. Independent of the origin behind the similarity, in a situation where it exists, it is beneficial that the method determines the human body gesture first available at the computer system of the orthopedic assistive device as the human body gesture associated with the detected myoelectric MES, such that the orthopedic assistive device can already begin performing the respective human body gesture. When, thereafter, the later one of the first human body gesture and the second human body gesture becomes available at the computer system and turns out to have a higher reliability measure associated therewith than the first available one of the first human body gesture and the second human body gesture, so that the human body gesture associated with the detected myoelectric signal is changed from the first available human body gesture to the later one, the orthopedic assistive device can change to performing the later human body gesture. Because of the similarity of the respective human body gestures, in many cases, this change from one human body gesture being performed by the orthopedic assistive device to the other results in only minor adjustments to the movement of the orthopedic assistive device. Those minor adjustments do not affect the safety and comfort of using the orthopedic assistive device, but improve the accuracy of its operation. In other words, changing from the first available human body gesture to the later one poses a refinement of the human body gesture associated to the detected MES, or to the movement performed by the orthopedic assistive device, respectively.

According to an embodiment, as soon as the first one of the first human body gesture and the second human body gesture is available at the computer system, said first one of the first human body gesture and the second human body gesture is determined as the human body gesture associated with the detected myoelectric signal.

According to some embodiments, the checking, using the computer system, for the response received using the wireless reception system may use a deferred synchronous communication and/or may use a future and/or may use a poll to check for the response received using the wireless reception system, in particular, wherein said poll may be performed when the computer system is not commanding a motor of the orthopedic assistive device to perform a movement and/or when the computer system does not comprise a pending command to command a motor of the orthopedic assistive device, in particular on a memory of the computer system.

Using deferred synchronous communication, futures, or polling mechanisms for checking responses optimizes the system's resource management. Polling when the system is not engaged in commanding a motor ensures that the device's computational resources are efficiently utilized, preventing interruptions in motor control and enhancing the device's overall performance. This feature provides a technical advantage by balancing communication checks with the operational demands of the orthopedic assistive device.

According to some embodiments, the orthopedic assistive device may be selected from one of the following: a prosthesis, an orthotic device, and an exoskeleton; in particular, the orthopedic assistive device may be a prosthesis, in particular, a hand prosthesis.

According to some embodiments, the recognition process to determine the second human body gesture corresponding to the detected myoelectric signal may be performed on the detected myoelectric signal or on the data derived from the detected myoelectric signal.

According to some embodiments, the data may be derived from the detected myoelectric signal and the derived data may be sent using the wireless transmission system before performing the recognition process to determine the second human body gesture corresponding to the detected myoelectric signal.

According to some embodiments, the method further comprises: receiving the sent, derived data at a remote computer system, the remote computer system being remote from the orthopedic assistive device; performing, using the remote computer system, an additional recognition process on the sent, derived data to determine the first human body gesture; and sending, using the remote computer system, the determined first human body gesture.

Performing an additional recognition process on the remote computer system leverages the potentially greater processing power and resources available remotely, enhancing the accuracy and speed of the method, providing a more responsive, reliable and safe operation of the orthopedic assistive device.

According to an embodiment, in the process step of sending the derived data using the wireless transmission system, the derived data are sent to the remote computer system using the wireless transmission system.

According to some embodiments, the process step of checking for the response received using the wireless reception system may refer to checking, using the wireless reception system, for a response received from the remote computer system using the wireless reception system.

According to some embodiments, the determined first human body gesture may be sent, using the remote computer system, to the computer system.

According to some embodiments, the remote computer system may be power-supplied from a power supply providing a larger electrical capacitance than a power supply from which the computer system is power-supplied. Alternatively, or in addition, the remote computer system may be power-supplied from grid power.

The computer system comprised in the orthopedic assistive device may be power-supplied from a battery comprised in the orthopedic assistive device, or comprised in the computer system, respectively.

According to some embodiments, the remote computer system being remote from the orthopedic assistive device may refer to or may comprise an absence of a wired data connection between the computer system and the remote computer system.

According to some embodiments, the additional recognition process on the sent, derived data to determine the first human body gesture corresponding to the detected myoelectric signal is performed using an additional convolutional neural network.

The additional convolutional neural network provides the advantages described above in the context of the convolutional neural network.

In respective embodiments, a fourth reliability measure may be calculated, based on the additional recognition process, using said additional convolutional neural network, and the method may further comprise sending, using the remote computer, the fourth reliability measure, in particular to the computer system.

According to some embodiments, said additional convolutional neural network is on the remote computer system.

According to some embodiments, said data are derived from the detected myoelectric signal using at least a first layer of said convolutional neural network, and the additional recognition process is performed on said data derived, using the at least first layer of said convolutional neural network, from the detected myoelectric signal.

In respective embodiments, said at least one first layer of said convolutional neural network may comprise at least one convolutional layer of said convolutional neural network and optionally further comprises at least one pooling layer of said convolutional neural network. Alternatively, or in addition, the data derived from the detected myoelectric signal may correspond to the output of said at least one first layer of said convolutional neural network.

Respective embodiments provide the advantages discussed above in the context of the embodiment wherein said data are derived from the detected myoelectric signal using at least a first layer of said convolutional neural network.

According to some embodiments, the method further comprises actuating a plurality of motors according to the human body gesture associated with the detected myoelectric signal.

In particular, the motors may be actuated according to the human body gesture associated with the detected myoelectric signal immediately when the human body gesture associated with the detected myoelectric signal has been determined.

The method may further comprise: when the human body gesture associated with the detected myoelectric signal is changed from the one of the first human body gesture and the second human body gesture which is first available at the computer system to the later one of the first human body gesture and the second human body gesture, actuating the plurality of motors according to the later one of the first human body gesture and the second human body gesture; in particular, immediately when the human body gesture associated with the detected myoelectric signal is changed from the one of the first human body gesture and the second human body gesture which is first available at the computer system to the later one of the first human body gesture and the second human body gesture.

Said motors may be comprised in the orthopedic assistive device.

The (plurality of) motors may be actuated to mimic the human body gesture associated with the detected myoelectric signal, in particular, to mimic the human body gesture associated with the detected myoelectric signal using said orthopedic assistive device.

The method may further comprise sending, by the computer system, a control command to at least one motor controller adapted to control a movement of said plurality of motors, said control command being based on the human body gesture associated with the detected myoelectric signal and being adapted to cause the motor controller to actuate the plurality of motors according to the human body gesture associated with the detected myoelectric signal, in particular, in embodiments wherein the motor controller is comprised in the orthopedic assistive device.

A second aspect provides an orthopedic assistive device comprising a computer system, the computer system comprising at least one processor, a wireless transmission system, and a wireless reception system. The computer system is adapted to: receive at least a part of a myoelectric signal as a detected myoelectric signal; and to perform the following first process steps: deriving data from the detected myoelectric signal, the derived data representing at least part of an information-content of the detected myoelectric signal, a data volume of the derived data being smaller than a data volume of the detected myoelectric signal; sending the derived data using the wireless transmission system; and checking for a response received using the wireless reception system, said response comprising a first human body gesture. The computer system is further adapted to perform, before, after, during, or in between the first process steps, using the computer system comprised in the orthopedic assistive device, a recognition process to determine a second human body gesture corresponding to the detected myoelectric signal; and to determine the one of the first human body gesture and the second human body gesture which is first available at the computer system as the human body gesture associated with the detected myoelectric signal.

According to various embodiments, the computer system of the orthopedic assistive device is further adapted to perform any of the operations described above in the context of the computer system of the orthopedic assistive device participating in the method according to the first aspect.

The orthopedic assistive device has the advantages explained above in the context of the method.

The orthopedic assistive device may further comprise an electronic measurement system adapted to measure a myoelectric signal on a human body, and to provide said myoelectric signal to the computer system for being received as the at least part of the myoelectric signal.

The orthopedic assistive device may further comprise a plurality of motors. The orthopedic assistive device may be adapted to actuate the plurality of motors according to the human body gesture associated with the detected myoelectric signal, in particular, to mimic the human body gesture associated with the detected myoelectric signal.

According to an embodiment, the orthopedic assistive device is one of the following: a prosthesis, an orthotic device, and an exoskeleton; in particular, the orthopedic assistive device is a prosthesis, such as a hand prosthesis.

A third aspect provides a client-server system comprising the orthopedic assistive device according to any of the embodiments described above. A client of the client-server system is provided or is provided at least in part by the computer system of the orthopedic assistive device. The client-server system further comprises a remote computer system being remote from the orthopedic assistive device. The remote computer system is adapted to perform the following process steps: receiving the sent, derived data; performing an additional recognition process on the sent, derived data to determine the first human body gesture; and sending the determined first human body gesture.

According to various embodiments, the remote computer system of the client-server system is further adapted to perform any of the operations described above in the context of the remote computer participating in the method according to the first aspect.

According to another aspect, a computer program comprises instructions which, when the computer program is executed by a computer, cause the computer to carry out the method according to any of the embodiments described above.

In particular, the computer program may comprise instructions which: when the computer program is executed by a computer system, cause the computer system to carry out the method according to any of the embodiments described above.

Alternatively, or in addition, the computer program may comprise instructions which: when the computer program is executed by a client-server system, cause the client-server system to carry out the method according to any of the embodiments described above.

According to a further aspect, a non-transitory computer readable storage medium has stored thereon the computer program according to any of the embodiments described above.

The computer program, or the non-transitory computer readable storage medium having the computer program stored thereon, respectively, provide the advantages described above in the context of the method.

### LIST OF FIGURES

In the following, a detailed description of the present disclosure and examples thereof are given with reference to the figures. The figures show schematic illustrations of
Fig. 1 to Fig. 6: the method according to different embodiments, each of Fig. 1 to Fig. 6 illustrating a different embodiment;
Fig. 7 to Fig. 9: an orthopedic assistive device according to different embodiments, each of Fig. 7 to Fig. 9 illustrating a different embodiment;
Fig. 10: an electronic measurement system adapted to measure a myoelectric signal on a human body;
Fig. 11: a client-server system according to an embodiment;
Fig. 12: a computer program according to an embodiment;
Fig. 13: a non-transitory computer readable storage medium having stored thereon the computer program;
Fig. 14a to Fig. 14c: a technique for detecting a myoelectric signal;
Fig. 15a to Fig. 15c: an alternative technique for detecting a myoelectric signal; and
Fig. 16: an orthopedic assistive device according to a different embodiment.

### DESCRIPTION OF EXAMPLES

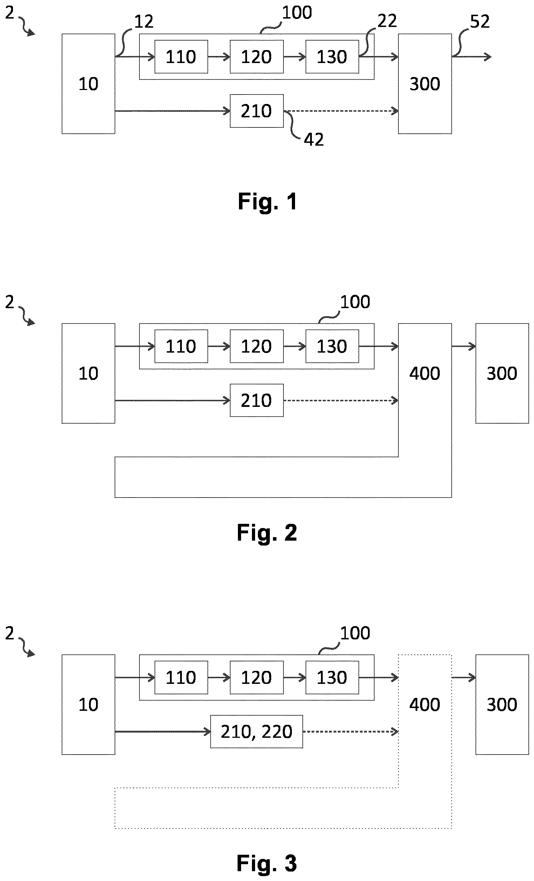

Fig. 1 illustrates an embodiment of a method 2 for associating a human body gesture 52 to a detected myoelectric signal 12. The method 2 comprises the following process steps: at step 10, detecting at least a part of a myoelectric signal as the detected myoelectric signal 12; first process steps 100; at step 210, performing a recognition process; and at step 300, determining the human body gesture 52 associated with the detected myoelectric signal 12.

In the following, the respective process steps are described in detail referring to an exemplary embodiment.

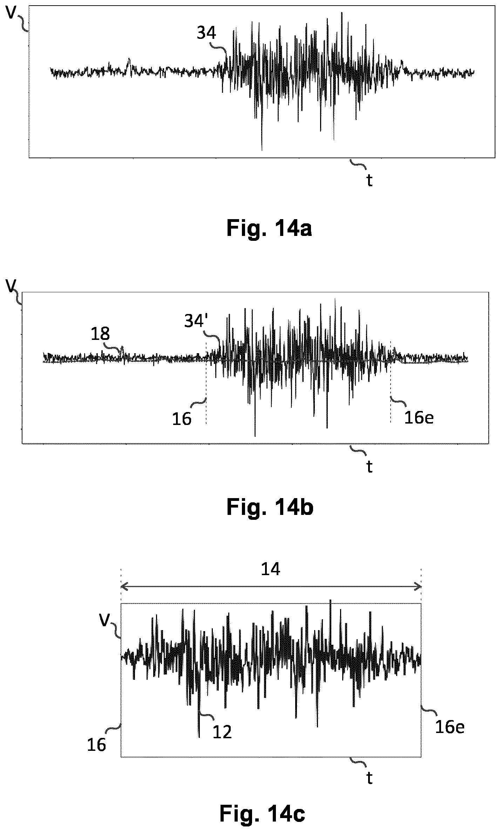

According to the exemplary embodiment of the method 2, a pair of electrodes is placed on different positions of a human body 70 at step 10. The voltage difference between the electrodes is measured using a low-noise, galvanically isolated, high-impedance (200 M Ohm) instrumentation amplifier. An exemplary, measured voltage difference is shown in Fig. 14a. The signal is filtered to remove mains hum (which typically occurs at 50 Hz or 60 Hz and at the respective harmonics) and to remove electrocardiographic signals, which originate from the muscle contractions of the heart muscle. Fig. 14b shows the resulting filtered voltage difference obtained from the measured voltage difference of Fig. 14a.

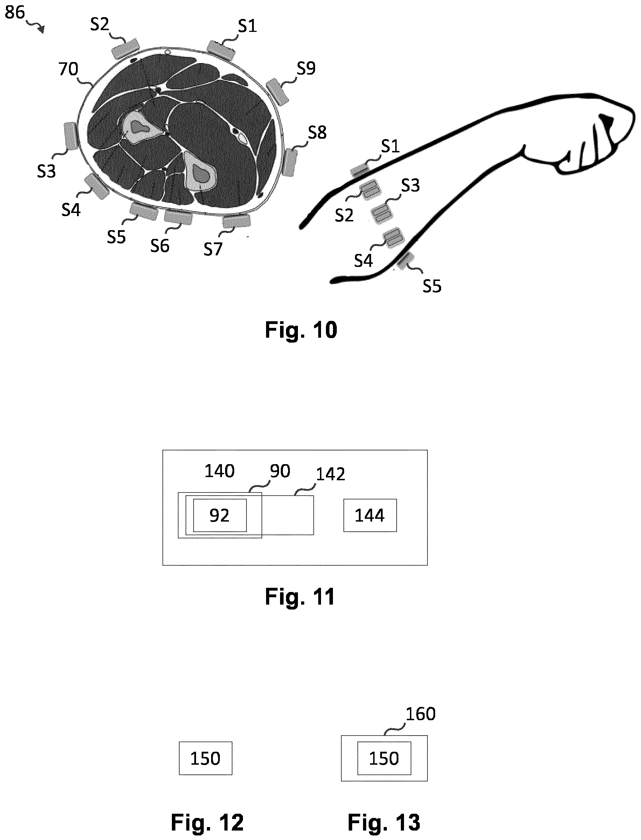

In preferred embodiments, more than one pair of electrodes is placed on different positions of a human body 70, as will be explained in detail in the context of Fig. 10.

Each of the first process steps 100 is performed using a computer system 92 comprised in an orthopedic assistive device 90. Said computer system 92 comprises at least one processor 94, a wireless transmission system 96, and a wireless reception system 98.

Typically, the computer system 92 further comprises a memory (not shown) communicatively coupled to the processor 94.

The respective computer system 92 is a battery-powered computer system 92, i.e., the computer system 92 comprises a battery for operating the computer system 92 for at least 1 h, preferably for several hours. This allows for carrying the computer system 92 by a user along with the orthopedic assistive device 90, independent of grid power supply, thus improving the versatility of the orthopedic assistive device 90. The computer system 92 is miniaturized (i.e., with a maximum weight of 150 g, preferably with a maximum weight of 100 g) to improve user comfort and to allow carrying it around along with the orthopedic assistive device 90.

The computer system 92 is comprised in the orthopedic assistive device 90 in a sense that a mechanical connection exists between the computer system 92 and the orthopedic assistive device 90, mechanically supporting the computer system 92 on the orthopedic assistive device 90, or attaching the computer system 92 to the orthopedic assistive device 90, respectively. Preferably, a wired connection exists between the computer system 92 and the orthopedic assistive device 90, facilitating wired communication between the computer system 92 and the orthopedic assistive device 90, in particular, with motors 82 thereof. The wired connection further allows for power-supplying the computer system 92 from the same power supply, for example from the same battery, as other parts of the orthopedic assistive device 90.

The first process steps 100 comprise deriving 110 data from the detected myoelectric signal 12, the derived data representing at least part of an information-content of the detected myoelectric signal 12, a data volume of the derived data being smaller than a data volume of the detected myoelectric signal 12; sending 120 the derived data using the wireless transmission system 96; and checking 130 for a response received using the wireless reception system 98, said response comprising a first human body gesture 22.

In other words, at step 110, the data volume of the detected myoelectric signal 12 is reduced for a subsequent process step of sending 120 the derived data using the wireless transmission system 96. The reduction of the data volume speeds up the subsequence sending 120, resulting in a reduced overall latency of the method 2.

According to some embodiments, in the process step of deriving 110 data from the detected myoelectric signal 12, the MES 12 is transformed into a spectrogram and optionally converted into Mel Frequency Cepstrum Coefficients. The resulting spectrogram, or the Mel Frequency Cepstrum Coefficients, respectively, is/are then used as the derived data. In respective embodiments, the detected MES 12 or data representing the detected MES 12, respectively, such as the spectrogram, or the Mel Frequency Cepstrum Coefficients, are preferably data-compressed within process step 110. Preferably, said data-compression is performed using an audio data compression technique, for example using an audio data compression technique based on psychoacoustics. The inventors have realized that myoelectric signals are similar to audio signals (in particular, regarding frequency range and the possibility for decomposition into carrier and envelope signals), and have successfully applied data compression techniques previously used for audio signals to the data compression of MES. In other words, audio compression techniques compress MES in a particularly efficient way.

According to an alternative, preferred embodiment, in the process step of deriving 110 data from the detected myoelectric signal 12, the computer system 92 executes at least a first layer (i.e., a convolutional layer) of a convolutional neural network. Even more preferably, in the process step of deriving 110 data from the detected myoelectric signal 12, the computer system 92 executes at least one convolutional layer and at least one pooling layer of a convolutional neural network. This embodiment has at least the following advantages: first, as the inventors have realized, the execution of the respective layers of a convolutional neural network is efficient in reducing the data volume of the data representing the detected MES 12, while maintaining a significant portion of its information content. Second, the data derived this way can again be used in concurrent step 210, and in particular in parts of step 210 subsequent to the process step of deriving 110 data from the detected myoelectric signal 12, as will be laid out in detail below, thus making twofold use of the deriving 110 of said data, or making efficient use of the limited computational capacity of the computer system 92, respectively.

The wireless transmission system 96 comprised in the computer system 92 and used for sending 120 the derived data is a Bluetooth, Wi-Fi, Zigbee, NFC, or cellular transmission system 96. A local (i.e., with a transmission distance below 30 m) wireless transmission system 96, also referred to as a local-area network, such as Bluetooth, Wi-Fi, Zigbee or NFC is preferred over the use of a wide-area (i.e., with a transmission distance above 30 m) wireless transmission system 96, also referred to as a wide-area network, such as a cellular transmission system 96, since the smaller transmission distance allows for operation at a lower power consumption.

Checking 130 for the response is performed using a deferred synchronous communication, meaning that the method proceeds with the subsequent process steps 210, 300 independent of whether a response has been received at step 130 or not.

In an exemplary embodiment, checking 130 for the response is performed by providing an inbox storage for a response to be received. For as long as a response has not yet been found by the checking 130, a poll is performed to check for the response in the inbox storage. Said poll is performed repeatedly for as long as a response has not yet been found in the inbox storage by the checking 130. Preferably, the poll is performed when the computer system 92 is not commanding a motor 82 of the orthopedic assistive device 90 to perform a movement, and the poll is preferably performed when a command to command a motor 82 of the orthopedic assistive device 90 is not pending on the computer system 92.

In an alternative embodiment, checking 130 for the response is performed using a future as described in Dean, M.A., Sands, R.E., Schantz, R.E.: "Canonical data representation in the Cronus distributed operating system". In: Proc. INFOCOM, pp. 814-819, IEEE (Apr 1987), which is incorporated herein by reference in its entirety, or as described in Gurwitz, R.F., Dean, M.A., Schantz, R.E.: "Programming support in the Cronus distributed operating system," In: Proc. 6th Int. Conf. on Distributed Computing Systems, pp. 486-493, IEEE (May 1986), which is incorporated herein by reference in its entirety, or as described in Liskov, B., Shrira, L.: "Promises: Linguistic support for efficient asynchronous procedure calls in distributed systems," ACM Sigplan Notices 23(7), 260-267 (1988), which is incorporated herein by reference in its entirety.

In an alternative embodiment, checking 130 for the response is performed using the method described in Iida, K., Kikuchi, J.: "Evaluation of a method for reliable message transfer communication in CORBA," In: Proc. 1st Int. Enterprise Distributed Object Computing Workshop. pp. 104-110 (1997), which is incorporated herein by reference in its entirety.

The description given above for the wireless transmission system 96 comprised in the computer system 92 and used for sending 120 the derived data applies correspondingly to the wireless reception system 98 comprised in the computer system 92 and used for receiving the response.

At step 210, the recognition process is performed before, after, during, or in between the first process steps 100. The recognition process is performed using the computer system 92 comprised in the orthopedic assistive device 90. The recognition process is performed to determine a second human body gesture 42 corresponding to the detected myoelectric signal 12.

According to an embodiment, the recognition process performed at step 210 uses a fuzzy logic which has been applied successfully in the context of audio recognition (i.e., a fuzzy audio search algorithm). More specifically, the recognition process according to an embodiment uses the algorithm described in Polo-Rodriguez, A.; Vilchez Chiachio, J.M.; Paggetti, C.; Medina-Quero, J.: "Ambient Sound Recognition of Daily Events by Means of Convolutional Neural Networks and Fuzzy Temporal Restrictions," Appl. Sci. 2021, 11, 6978, {https://doi.org/10.3390/app11156978}, in the following also referred to as Polo-Rodriguez et at., and more specifically in Section 2.2 ("Fuzzy Protoforms to Describe Daily Events from Audio Recognition Streams") therein.

According to an embodiment, which uses in the recognition process performed at step 210 a modification of the aforementioned algorithm from Polo-Rodriguez et at., the detected MES 12 is provided from a beginning 16 to an end 16e of a non-resting phase (as described in more detail in the context of Fig. 14a to Fig. 14c), and in a fuzzification step, the signal is mapped onto a fuzzy variable based on classifiers for MES known from the prior art, or from expert knowledge, respectively. These classifiers include, according to different embodiments, one or any combination of the following: the number of zero crossings in the series of filtered voltage difference values V_i, the amplitude (i.e., the maximum) of the filtered voltage difference values V_i, the root mean square of the filtered voltage difference values V_i, autoregressive coefficients determined from the filtered voltage difference values V_i, and an approximate entropy determined from the filtered voltage difference values V_i. According to some embodiments, the method is a method for controlling a prosthesis, the selection of a specific classifier or combination of classifiers is made by an expert depending on the properties of the prosthesis (e.g., body part that the prosthesis corresponds to) and the physical condition (e.g., presence of muscle structure in the proximity of the prosthesis) of the human being on which the myoelectric signal is detected.

Based on experience, the detected MES 12 is mapped, according to an embodiment, onto a first fuzzy variable taking the form (zero crossings_many, zero crossings_average, zero crossings_few, amplitude_small, amplitude_average, amplitude_large). Herein, "zero crossings_many" represents a value quantifying to what degree the number of zero crossings in the series of filtered voltage difference values V_i is large; "zero crossings_average" represents to what degree it is average; and "zero crossings_ few" represents to what degree it is small. Correspondingly, "amplitude_small" represents a value quantifying to what degree the maximum in the filtered voltage difference values V_i is small; "amplitude_average" represents to what degree it is average; and "amplitude_large" represents to what degree it is large. The mapping is done according to expert knowledge known from the prior art.

In a fuzzy inference step, the first fuzzy variable is converted into a second fuzzy variable, which represents the human body gestures to be identified, which include, but are not limited to: open hand, closed fist, flexion, extension, supination. In other words, according to an example, the second fuzzy variable takes the form ("value_open hand", "value_closed fist", "value_flexion", "value_extension", "value_supination"). The value "value_open hand" represents to what degree the detected MES 12 represents an open hand, based on the fuzzy inference which determines the value "value_open hand" according to a fuzzy logic applied to the first fuzzy variable (i.e., the values "zero crossings_many", "zero crossings_average",...) as an input. The conversion, i.e., the fuzzy inference, is performed according to expert knowledge known from the prior art. Correspondingly, the value "value_closed fist" represents to what degree the detected MES 12 represents a closed fist according to said fuzzy logic, and so on.

In a further, defuzzification step, the second human body gesture 42 is derived from the second fuzzy variable, for example, as the human body gesture for which the corresponding value in the second fuzzy variable is the largest.

According to an alternative embodiment, the recognition process performed at step 210 uses an Evolving Fuzzy Neural Network, as described, e.g., in Malcangi, M.: "Fuzzy logic-based audio pattern recognition," in: AIP Conference Proceedings, vol. 1060, pp. 225-228, American Institute of Physics (2008).

According to an alternative embodiment, the recognition process performed at step 210 uses a Gaussian Mixture Modell, as described, for example, in Vuegen, L., Broeck, B.V.D., Karsmakers, P., Gemmeke, J.F., Vanrumste, B., hamme, H.V.: "An mfcc-gmm approach for event detection and classification," in: Proceedings of the IEEE Workshop Appl. Signal Process, Audio Acoust. (2013).

According to an alternative embodiment, the recognition process performed at step 210 uses a Hidden Markov Model, as described, for example, in M. Gales and S. Young, "The Application of Hidden Markov Models in Speech Recognition," Foundations and TrendsR in Signal Processing, vol 1, no 3, pp 195-304, 2007 {http://dx.doi.org/10.1561/2000000004}.

In an alternative embodiment, the recognition process performed at step 210 uses an algorithm similar to the one described in Wang, A.: "An industrial strength audio search algorithm," in: Proceedings of the 4th International Conference on Music Information Retrieval (ISMIR 2003), Baltimore, MD, USA, October 27-30, 2003 (2003), which, in the following, is also referred to as Wang et al. This algorithm calculates hashes from both the input data, i.e., the detected MES 12 in the current description, and from database records, and compares the respective hashes. The hashes are generated by first converting each of the respective data (i.e., the reference data from a database comprising said database records as well as the data on which the recognition process is to be performed) into a spectrogram. In other words, the respective signals are converted into the time-frequency-domain. Subsequently, peaks are identified in the spectrogram. Then, any pair of peaks is identified, for which the distance between the respective peaks of the pair does not exceed a predefined distance in the spectrogram (or in the time-frequency-domain, respectively). For each peak pair, a corresponding hash is stored. The hash contains the following values: frequency of the first peak of the pair, frequency of the second peak of the pair, and time difference between the peaks of the pair. The hash is annotated with one of the times associated with the peaks (e.g., the minimum of the times associated with the peaks of the pair). The algorithm searches for matches between hashes of the input data and hashes in the database, i.e., of the database records.

A further embodiment is similar to the one described in the previous paragraph, but in addition to the frequency of the first peak of the pair, the frequency of the second peak of the pair, and the time difference between the peaks of the pair, the hash further comprises the amplitude of the first peak of the pair, and the amplitude of the second peak of the pair, on a linear scale or in the form of a Mel Frequency Cepstrum Coefficient.

Notably, the algorithm of Wang et al. finds a match when the hashes of the input data and the hashes in the database, i.e., of the database records, are identical. In an embodiment of the current disclosure, this algorithm is modified to find a match if the difference between the corresponding values contained in said hashes is smaller or equal to a predefined value. Said difference is further used to calculate a similarity metric between the detected MES 12 and the database record that one of the hashes corresponds to.

All the embodiments described in the foregoing paragraphs have in common, that they apply at step 210 a recognition process which has previously proven successful for audio search and recognition. The inventors have realized that such algorithms are particularly suitable for the gesture recognition based on MES. The aforementioned embodiments make use of this realization to provide a method 2 with enhanced MES classification accuracy.

In the context of the embodiments described in the foregoing paragraphs, a database has been described. Said database comprises a plurality of database records, each of said database records comprising a reference myoelectric signal and a corresponding reference human body gesture associated thereto.

To obtain the database, filtered voltage differences like the one described above and in the context of Fig. 14b are provided as the reference myoelectric signals, and are annotated with a corresponding, associated human body gesture (reference human body gesture). The respective annotated filtered voltage differences are obtained by instructing a human being to perform a specific human body gesture. As the human being performs the specific human body gesture, the filtered voltage difference is acquired/measured as described above in the context of step 10. The resulting data are stored as a series of data pairs of timestamps t_i and filtered voltage difference values V_i, representing the reference myoelectric signal, and are annotated with the specific human body gesture. Possible values/classes for the specific human body gestures include, but are not limited to: open hand, closed fist, flexion, extension, supination.

For the sake of clarity/simplicity, the acquisition and processing of the reference myoelectric signal has been described using a single pair of electrodes. However, in preferred embodiments, multiple pairs of electrodes are applied to the human body during the acquisition, to obtain multiple series of voltage differences and filtered voltage difference values V1_i, V2_i, V3_i,... in parallel, as the reference myoelectric signal, as explained in detail below in the context of Fig. 10. In such embodiments, the reference myoelectric signal comprises, or is based on (with the described processing of the MES applied to each series of voltage differences) the multiple series of voltage differences and filtered voltage difference values V1_i, V2_i, V3_i,....

The acquisition of the reference myoelectric signal is performed for a plurality of specific gestures, wherein, for each of the specific gestures, the human being is instructed to perform the respective specific human body gesture several times (e.g., 100 times), and each time the filtered voltage difference is acquired as the human being performs the respective specific human body gesture, to obtain the reference myoelectric signal. All resulting reference myoelectric signals and the corresponding, annotated reference human body gesture together form the database.

As an alternative to the embodiments described above, wherein at step 210 a recognition process is applied which has previously proven successful for audio search and recognition, the recognition process performed at step 210, according to some embodiments, uses one or at least one of the following: LinearDiscriminant, QuadraticDiscriminant, SubspaceDiscriminant, SVMLinear, SVMQuadratic, SVMCubic, SVMFineGaussian, SVMMediumGaussian, SVMCoarseGaus, SimpleTree, MediumTree, ComplexTree, BaggedTree, BoostedTree, RUSBoostedTrees, KNNFine, KNNMedium, KNNCoarse, KNNCubic, KNNCosine, KNNWeighted, SubSpaceKNN, a forward neural network, recur6, and a convolutional neural network.

As a further alternative to the embodiments described above, wherein at step 210 a recognition process is applied which has previously proven successful for audio search and recognition, in the recognition process performed at step 210, according to some embodiments, a machine learning model is applied (i.e., the machine learning model is applied in performing the recognition process to determine the second human body gesture 42).

In respective embodiments, the training of the machine learning model is performed using the database described above.

According to an embodiment, the series of data pairs of timestamps t_i and filtered voltage difference values V_i is directly used as training data for training the machine learning model, together with the annotated specific human body gesture.

According to another embodiment, a beginning 16 and an end 16e of a non-resting phase is detected in the series of data pairs of timestamps t_i and filtered voltage difference values V_i as described in detail below, and the series of data pairs of timestamps t_i and filtered voltage difference values V_i from the beginning 16 to the end 16e of the non-resting phase is used as training data for training the machine learning model, together with the annotated specific human body gesture.

According to yet other embodiments, the series of data pairs of timestamps t_i and filtered voltage difference values V_i, in total or only from the beginning 16 to the end 16e of the non-resting phase, is transformed into a spectrogram and optionally converted into Mel Frequency Cepstrum Coefficients, and the resulting spectrogram or the Mel Frequency Cepstrum Coefficients are used as training data for training the machine learning model, together with the annotated specific human body gesture.

When the recognition process is performed at step 210 using the so-trained machine learning model, the detected myoelectric signal 12 is prepared like the myoelectric signals used for training the machine learning model. In other words, in embodiments, wherein the machine learning model is trained with the series of data pairs of timestamps t_i and filtered voltage difference values V_i as training data, a series of data pairs of timestamps t_i and filtered voltage difference values V_i obtained at step 10 is directly used as the detected myoelectric signal 12. In embodiments, wherein the machine learning model is trained with the data from the beginning 16 to the end 16e of a non-resting phase, the detected myoelectric signal 12 only uses data from the beginning 16 to the end 16e of a non-resting phase. In embodiments, wherein the myoelectric signals used as training data have been converted into spectrograms, the detected MES 12 is converted into a spectrogram also for the recognition process at step 210, etc.

Preferably, the applied machine learning model is a convolutional neural network.

The inventors have systematically compared the rate of correct identification of the human body gesture 52 using recognition processes based on LinearDiscriminant, QuadraticDiscriminant, SubspaceDiscriminant, SVMLinear, SVMQuadratic, SVMCubic, SVMFineGaussian, SVMMediumGaussian, SVMCoarseGaus, SimpleTree, MediumTree, ComplexTree, BaggedTree, BoostedTree, RUSBoostedTrees, KNNFine, KNNMedium, KNNCoarse, KNNCubic, KNNCosine, KNNWeighted, SubSpaceKNN, a forward neural network, recur6, and a convolutional neural network. The convolutional neural network has been found to outperform any of the other approaches.

Applying a convolutional neural network in the recognition process performed at step 210 further has the advantage that, as a part of said application of the convolutional neural network, the initial layer of the convolutional neural network, namely one or several convolution layer(s) and a subsequent at least one pooling layer, generate data with a reduced data volume as compared to the detected MES 12. These data, which are derived from the detected MES 12, are, in a preferred embodiment of the method 2, extracted from the convolutional neural network after the initial layer(s) of the convolutional neural network, and used as the derived data being sent at step 120.

In other words, in some embodiments, the sending 120 is performed after performing 210 a portion of the recognition process to determine the second human body gesture 42 corresponding to the detected myoelectric signal 12. In respective embodiments, performing 210 said portion of the recognition process comprises applying an initial layer or initial layers of a convolutional neural network applied in the recognition process performed at step 210.

Respective embodiments make twofold use of applying the initial layers of the convolutional neural network, both for deriving the data to be sent, and also for the recognition process performed at step 210. They thus make improved use of the limited computational resources of the computer system 92.

At step 300, the one of the first human body gesture 22 and the second human body gesture 42 which is first available at the computer system 92 is determined, by the computer system 92, as the human body gesture 52 associated with the detected myoelectric signal 12.

In the depicted example, the first human body gesture 22 is first available at the computer system 92, and is thus determined as the human body gesture 52 associated with the detected myoelectric signal 12. For this reason, the corresponding arrow is depicted as a continuous line in Fig. 1. The second human body gesture 42 only becomes available later at the computer system 92, i.e., it is the later one of the first and second human body gestures 22, 42. For this reason, the corresponding arrow is depicted as a dashed line in Fig. 1.

In other examples (not shown), the second human body gesture 42 is first available at the computer system 92, and the first human body gesture 22 is the later one of the first human body gesture 22 and the second human body gesture 42. Whether one or the other situation occurs, depends, for example on the quality of the wireless communication for the sending and reception at steps 120, 130, and on the availability of a server to provide the first human body gesture 22.

The method 2 ensures that the orthopedic assistive device 90 can start performing a movement as soon as either of the first human body gesture 22 and the second human body gesture 42 is available at the computer system 92. In other words, it ensures a fast response of the orthopedic assistive device 90 and avoids unnecessary idling of the orthopedic assistive device 90.

In particular, the one of the first human body gesture 22 and the second human body gesture 42 which is first available at the computer system 92 is determined, by the computer system 92, as the human body gesture 52 associated with the detected myoelectric signal 12 independent of whether the checking 130 has received a response using the wireless reception system 98. Thus, for example in case of a failure of a server or in case of poor wireless reception, the orthopedic assistive device 90 can start performing a movement as soon as the second human body gesture 42 is available at the computer system 92.

Fig. 2 shows an embodiment of a method 2 similar to the one of the foregoing embodiment. Similar elements and method steps are indicated by same reference numerals. For the sake of brevity and to avoid repetition, they will not be described again, but reference is made to the respective description above. In the following, the description of Fig. 2 will be limited to modifications over the embodiment of Fig. 1.

The method 2 according to the embodiment of Fig. 2 comprises the additional process step of checking 400, by the computer system 92, if a first reliability measure is available at the computer system 92. If the first reliability measure is available at the computer system 92 and exceeds a first threshold reliability measure value, the one of the first human body gesture 22 and the second human body gesture 42 which is first available at the computer system 92 is determined as the human body gesture 52 associated to the detected myoelectric signal 12. Preferably, the one of the first human body gesture 22 and the second human body gesture 42 which is first available at the computer system 92 is determined as the human body gesture 52 associated to the detected myoelectric signal 12 only if the first reliability measure is available at the computer system 92 and exceeds a first threshold reliability measure value.

The first reliability measure that is checked for is associated with the one of the first human body gesture 22 and the second human body gesture 42 which is first available at the computer system 92.

The first reliability measure that is checked for is associated with a likelihood for said one of the first human body gesture 22 and the second human body gesture 42 which is first available at the computer system 92 to correspond to the detected myoelectric signal 12.

The additional process step 400 ensures that the gesture identified by the method 2 is accurate and corresponds to the detected myoelectric signal 12. For this purpose, the reliability measure is compared against a threshold value to determine its validity. According to different embodiments, this first threshold reliability measure value amounts to 40% or 50% or 60% or 70%. Considering the reliability measure helps to avoid situations wherein the orthopedic assistive device 90 would receive an incorrectly assigned human body gesture 52 and would perform a movement according to this incorrectly assigned human body gesture 52, which could affect the safety and comfort of using the orthopedic assistive device 90. The additional step 400 thus improves the safety and reliability of the method 2 and of the orthopedic assistive device 90.

In the depicted embodiment, the recognition process at step 210 is performed using a convolutional neural network.

As is known to the skilled person from common general knowledge, a convolutional neural network outputs a reliability measure indicating the likelihood of the classification result to be correct, typically in the form of a softmax probability. The higher the softmax probability, the higher is the likelihood for the human body gesture 52 that has been determined using the convolutional neural network to correspond to the detected myoelectric signal 12. The reliability measure output by the convolutional neural network is thus beneficially applied in the method according to any of the embodiments for at least one or all of the described reliability measures. More specifically, in respective embodiments, the recognition process (i.e., performed by the computer system 92) to determine the second human body gesture 42 corresponding to the detected myoelectric signal 12 is performed using a convolutional neural network, and the reliability measure output by said convolutional neural network is used as the second reliability measure and, if the second human body gesture 42 is first available at the computer system 92, also as the first reliability measure, or, if the second human body gesture 42 is the later one of the first human body gesture 22 and the second human body gesture available at the computer system 92, as the third reliability measure. In preferred embodiments, the respective (first or second or third) reliability measure is compared against the respective (first or second or third) threshold reliability measure value (e.g., according to the exemplary values given above). If - and, in particular, only if - the respective (first or second or third) reliability measure exceeds the corresponding threshold reliability measure value, the second human body gesture 42 is determined as the human body gesture 52 associated with the detected myoelectric signal 12. Otherwise, the second human body gesture 42 is rejected, i.e., from being determined as the human body gesture 52 associated with the detected myoelectric signal 12. Respective embodiments improve the safety of operation of the computer-implemented method 2 and of the orthopedic assistive device 90, since a second human body gesture 42 with a low associated reliability measure, which, with some probability, has not been the gesture intended by the user, is not performed. Alternatively, or in addition, the recognition process (i.e., performed by the remote computer system 144) to determine the first human body gesture 22 corresponding to the detected myoelectric signal 12 is performed using an additional convolutional neural network, and the reliability measure output by the additional convolutional neural network is associated with the first human body gesture 22. In respective embodiments, said reliability measure output by the additional convolutional neural network and associated with the first human body gesture 22 is used as the fourth reliability measure and, if the first human body gesture 22 is first available at the computer system 92, also as the first reliability measure or, if the first human body gesture 22 is the later one of the first human body gesture 22 and the second human body gesture available at the computer system 92, as the third reliability measure. The further comparison against an associated threshold reliability value (i.e., a fourth or first or third threshold reliability value associated with the fourth or first or third reliability measure) and the subsequent conditional determining of the human body gesture 52 depending on the outcome of the comparison is then performed as described above for the reliability measure output by the convolutional neural network applied in the recognition process performed using the computer system 92 to determine the second human body gesture 42 corresponding to the detected myoelectric signal 12.

Notably, if a response is received using the wireless reception system 98 at step 130, the first human body gesture 22 comprised in the response, according to some embodiments, has also been obtained using a convolutional neural network. Respective embodiments provide advantages similar to the ones described above in the context of the convolutional neural network used in the recognition process performed at step 210.

Process step 400 has been described in the context of the embodiment of Fig. 2. However, performing process step 400 is not limited to that embodiment. Preferably, process step 400 is performed in the method 2 of any of the embodiments of Fig. 1, Fig. 3, Fig. 4, Fig. 5, or Fig. 6.

Fig. 3 shows an embodiment of a method 2 similar to the one of the foregoing embodiments. Similar elements and method steps are indicated by same reference numerals. For the sake of brevity and to avoid repetition, they will not be described again, but reference is made to the respective description above. In the following, the description of Fig. 3 will be limited to modifications over the embodiments of Fig. 1 and Fig. 2.

As an optional process step, the method 2 according to the embodiment of Fig. 3 preferably comprises step 400 as described above. Since method step 400 is optional in this embodiment, it is indicated with dashed lines in Fig. 3.

As compared to the method 2 according to the embodiments described above, the method 2 of Fig. 3 comprises the further process step of calculating 220, using the computer system 92, based on the recognition process 210 to determine the second human body gesture 42 corresponding to the detected myoelectric signal 12, a second reliability measure, the second reliability measure being associated with a likelihood for the second human body gesture 42 to correspond to the detected myoelectric signal 12. If the one of the first human body gesture 22 and the second human body gesture 42 which is first available at the computer system 92 is the second human body gesture 42, the second human body gesture 42 is determined as the human body gesture 52 associated to the detected myoelectric signal 12, if the second reliability measure exceeds a second threshold reliability measure value, and preferably only if the second reliability measure exceeds the second threshold reliability measure value.

The exemplary values given above for the first threshold reliability measure value apply correspondingly to the second threshold reliability measure value.

The consideration of the second threshold reliability measure brings advantages similar to the advantages explained above in the context of the first threshold reliability measure.

In the depicted embodiment, the recognition process at step 210 is performed using a convolutional neural network. As explained above, a convolutional neural network outputs a reliability measure indicating the likelihood of the classification result to be correct, typically in the form of a softmax probability. In the embodiment of Fig. 3, this reliability measure is used as the second threshold reliability measure.

Process step 220 has been described in the context of the embodiment of Fig. 3. However, performing process step 220 is not limited to that embodiment. Preferably, process step 220 is performed in the method 2 of any of the embodiments of Fig. 1, Fig. 2, Fig. 4, Fig. 5, or Fig. 6.

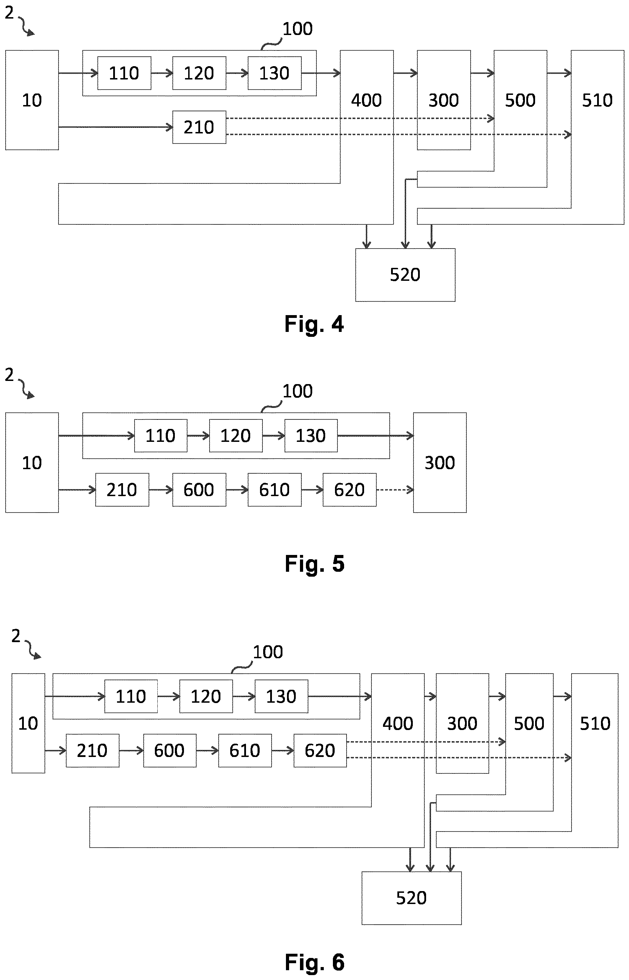

Fig. 4 shows an embodiment of a method 2 similar to the one of the foregoing embodiments. Similar elements and method steps are indicated by same reference numerals. For the sake of brevity and to avoid repetition, they will not be described again, but reference is made to the respective description above. In the following, the description of Fig. 4 will be limited to modifications over the embodiments of Fig. 1, Fig. 2, and Fig. 3.

As compared to the method 2 according to the foregoing embodiments, the method 2 of Fig. 4 further comprises the process step of checking 500, by the computer system 92, if a later one of the first human body gesture 22 and the second human body gesture 42 is available at the computer system 92.

In other words, the method 2, or the classification process, respectively, does not terminate when the first one of the first human body gesture 22 and the second human body gesture 42 has become available at the computer system 92. Instead, the computer system 92 continues checking for the other one of the first and second human body gesture 22, 42 to become available.

Said checking 500 is performed similarly to the checking 130 described above in detail, in particular, using the poll described above in detail or using the future described above in detail or using the method of Iida, K., Kikuchi, J.: "Evaluation of a method for reliable message transfer communication in CORBA," In: Proc. 1st Int. Enterprise Distributed Object Computing Workshop. pp. 104-110 (1997).

As compared to the method 2 according to the foregoing embodiments, the method 2 of Fig. 4 further comprises the process step of checking 510, by the computer system 92, if a third reliability measure associated with the later one of the first human body gesture 22 and the second human body gesture 42 is available at the computer system 92, the third reliability measure being associated with a likelihood for said later one of the first human body gesture 22 and the second human body gesture 42 to correspond to the detected myoelectric signal 12.

Typically, the third reliability measure is received together with the later one of the first human body gesture 22 and the second human body gesture 42. The checking 510 is thus performed similarly to the checking 500 described above.

As compared to the method 2 according to the foregoing embodiments, the method 2 of Fig. 4 further comprises the process step of, if the first reliability measure, the third reliability measure and the later one of the first human body gesture 22 and the second human body gesture 42 are available at the computer system 92, and if the third reliability measure exceeds the first reliability measure: changing 520 the human body gesture 52 associated with the detected myoelectric signal 12 from the one of the first human body gesture 22 and the second human body gesture 42 which is first available at the computer system 92 to the later one of the first human body gesture 22 and the second human body gesture 42.

Thus, when the later one of the first/second human body gesture 22, 42 becomes available at the computer system 92, it is used for a refinement of the human body gesture 52 associated to the detected MES 12, or of the movement performed by the orthopedic assistive device 90, respectively.

The exemplary values given above for the first threshold reliability measure value apply correspondingly to the third threshold reliability measure value.

In some embodiments, the human body gesture 52 associated with the detected myoelectric signal 12 is changed from the one of the first human body gesture 22 and the second human body gesture 42 which is first available at the computer system 92 to the later one of the first human body gesture 22 and the second human body gesture 42 only if a time delay between a first moment in time when the detected myoelectric signal 12 is received at the computer system 92 and a second moment in time when the checking 500 if the later one of the first human body gesture 22 and the second human body gesture 42 is available at the computer system 92 has found that the later one of the first human body gesture 22 and the second human body gesture 42 is available at the computer system 92 does not exceed a threshold time delay. In some embodiments, the threshold time delay is associated with a duration of the human body gesture 52 associated with the detected myoelectric signal 12. In exemplary embodiments, the threshold time delay corresponds to half of said duration or to 70% of said duration or to 90% of said duration or to said duration.

Respective embodiments prevent that, when the human body gesture 52 has already been executed for the most part, the orthopedic assistive device 90 switches to another human body gesture and starts performing the latter, which might be irritating to the user. Respective embodiments thus improve reliability and comfort of operating the orthopedic assistive device 90.

Process steps 500, 510, and 520 have been described in the context of the embodiment of Fig. 4. However, performing process steps 500, 510, and 520 is not limited to that embodiment. Preferably, process steps 500, 510, and 520 are performed in the method 2 of any of the embodiments of Fig. 1, Fig. 2, Fig. 3, Fig. 5, or Fig. 6.

Fig. 5 shows an embodiment of a method 2 similar to the one of the foregoing embodiments. Similar elements and method steps are indicated by same reference numerals. For the sake of brevity and to avoid repetition, they will not be described again, but reference is made to the respective description above. In the following, the description of Fig. 5 will be limited to modifications over the embodiments of Fig. 1, Fig. 2, Fig. 3 and Fig. 4.

As compared to the method 2 according to the foregoing embodiments, the method 2 of Fig. 5 further comprises the process step of receiving 600 the sent, derived data at a remote computer system 144, the remote computer system 144 being remote from the orthopedic assistive device 90.

In the depicted embodiment, the remote computer system 144 is a battery-powered laptop with a high computing power, i.e., as compared to the low computing power of the computer system 92 of the orthopedic assistive device 90. The laptop is remote in a sense that it does not have any mechanical connection to the orthopedic assistive device 90 and does not have any wired connection to the orthopedic assistive device 90 allowing for a communication via said wired connection. Instead, the laptop communicates with the computer system 92 of the orthopedic assistive device 90 through a wireless reception and transmission system of the laptop, improving the mobility and versatility of the orthopedic assistive device 90. The laptop provides a convenient and cost-efficient way to establish a remote computer system 144 with a high computing power, for example at the home or at the workplace of the user of the orthopedic assistive device 90.

In an alternative embodiment, the remote computer system 144 is a battery-powered smartphone, i.e., a system with a wireless reception and transmission system for communication with the computer system 92 of the orthopedic assistive device 90, with at least one processor for providing the high computing power, and with a weight of no more than 300 g, preferably of no more than 200 g.

The description given above in the context of the wireless transmission system 96 and the wireless reception system 98 applies correspondingly to the wireless reception and transmission system of the remote computer system 144.

In an alternative embodiment, the remote computer system 144 is the Internet, and access of the computer system 92 of the orthopedic assistive device 90 to the remote computer system 144 is facilitated through a WLAN router located in the vicinity of the orthopedic assistive device 90.

As compared to the method 2 according to the foregoing embodiments, the method 2 of Fig. 5 further comprises the process step of performing 610, using the remote computer system 144, an additional recognition process on the sent, derived data to determine the first human body gesture 22.

The additional recognition process is performed similarly to the recognition process of step 210 described in detail above. In other words, the detailed description above of the recognition process of step 210 applies correspondingly to the additional recognition process.

Notably, due to the high computational power of the remote computer system 144, the additional recognition process is, according to some embodiments, performed faster than the recognition process of step 210.

Making use of the high computational power of the remote computer system 144, the additional recognition process applies, according to some embodiments, a higher-accuracy technique than the recognition process of step 210. For this purpose, according to some embodiments, in the additional recognition process, a database larger than the database used in the recognition process of step 210 is applied, or a machine learning model, in particular a convolutional neural network, with a larger number of nodes and/or parameters is used in the additional recognition process as compared to the number of nodes and/or parameters of the machine learning model, or of the convolutional neural network, respectively, used in the recognition process of step 210.

As compared to the method 2 according to the foregoing embodiments, the method 2 of Fig. 5 further comprises the process step of sending 620, using the remote computer system 144, the determined first human body gesture 22.

More specifically, the first human body gesture 22 determined in the additional recognition process is sent using the wireless reception and transmission system of the remote computer system 144 to the computer system 92 of the orthopedic assistive device 90, which receives the first human body gesture 22 via its wireless reception system 98.

The use of the remote computer system 144 having a high computational power improves the accuracy of the method 2.

Process steps 600, 610, and 620 have been described in the context of the embodiment of Fig. 5. However, performing process steps 600, 610, and 620 is not limited to that embodiment. Preferably, process steps 600, 610, and 620 are performed in the method 2 of any of the embodiments of Fig. 1, Fig. 2, Fig. 3, Fig. 4, or Fig. 6.

Fig. 6 shows an embodiment of a method 2 similar to the one of the foregoing embodiments. Similar elements and method steps are indicated by same reference numerals. For the sake of brevity and to avoid repetition, they will not be described again, but reference is made to the respective description above. In the following, the description of Fig. 6 will be limited to modifications over the embodiments of Fig. 1, Fig. 2, Fig. 3, Fig. 4 and Fig. 5.

The method 2 according to the embodiment of Fig. 6 comprises process step 400 described in detail above in the context of Fig. 2. The method 2 according to the embodiment of Fig. 6 further comprises process steps 500, 510, and 520 described above in detail in the context of Fig. 4. The method 2 according to the embodiment of Fig. 6 further comprises process steps 600, 610, and 620 described above in detail in the context of Fig. 5.

A respective combination of process steps is particularly beneficial, since they act together to provide both the first and the second human body gesture 22, 42 at steps 210, 600, 610, 620, a judgment of the reliability of the first one of said first and second human body gestures 22, 42 at step 400, and a refinement of the determined human body gesture 52 at steps 500, 510, 520. The method 2 according to the respective embodiment is particularly efficient when convolutional neural networks are applied in the recognition processes at steps 210, 610, as described in detail below.

In the depicted embodiment, the recognition process performed at step 210 uses a first convolutional neural network. The additional recognition process performed at step 610 uses an additional convolutional neural network. The additional convolutional neural network is preferably larger than the first convolutional neural network, for example in terms of its number of nodes and/or in terms of its number of parameters and/or in terms of a size of the database with training data that the respective convolutional neural networks have been trained on.

In the depicted embodiment, the convolutional neural network used in the recognition process performed at step 210 is used to derive the data with the data volume smaller than the data volume of the detected myoelectric signal 12, as described in detail above in the context of the embodiment wherein the convolutional neural network is applied in the recognition process performed at step 210.

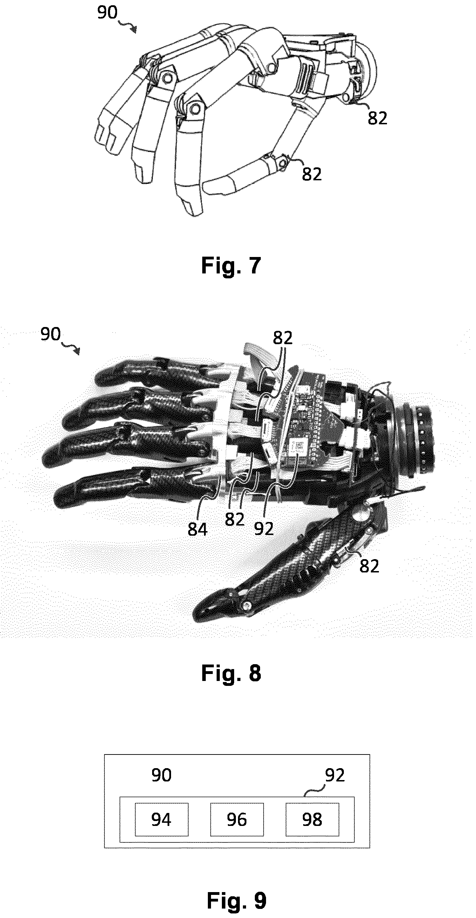

Fig. 7 illustrates an orthopedic assistive device 90.

The exemplary orthopedic assistive device 90 is a prothesis 90, and more specifically, a hand prosthesis 90.

The hand prosthesis 90 provides several degrees of freedom implemented with axes, the axes corresponding to finger segments, metacarpals, etc., and joints connecting said axes. Said degrees of freedom are adapted to mimic the degrees of freedom of a human hand.

According to an embodiment, when a human body gesture 52 associated to a detected myoelectric signal 12 is determined using the method 2 described above, the motors 82 are actuated to move the hand prosthesis 90 such that its movement mimics the human body gesture 52.

According to an alternative embodiment, when a human body gesture 52 associated to a detected myoelectric signal 12 is determined using the method 2 described above, the motors 82 are actuated to move the hand prosthesis 90 such that its movement mimics a pre-stored gesture associated with the human body gesture 52 according to a gesture database. Respective embodiments are particularly beneficial in situations, where a human being wearing the hand prosthesis 90 does not have all the muscles of a healthy subjects (as indicated, for example, in Fig. 10) and is unable to perform, with his/her muscles, the gesture that the hand prosthesis 90 is supposed to carry out.

Fig. 8 illustrates an orthopedic assistive device 90 according to another embodiment, similar to the embodiment of Fig. 7. Similar elements and method steps are indicated by same reference numerals. For the sake of brevity and to avoid repetition, they will not be described again, but reference is made to the respective description above. In the following, the description of Fig. 8 will be limited to modifications over the embodiment of Fig. 7.

In Fig. 8, as compared to Fig. 7, the computer system 92 of the hand prosthesis 90 is further indicated.

In Fig. 8, as compared to Fig. 7, a motor controller 84 of the hand prosthesis 90 is further indicated. The motor controller 84 is adapted to control a movement of the motors 82 of the hand prosthesis 90 in response to a command received from the computer system 92.

In alternative embodiments (not shown) several motor controllers 84 are comprised in the orthopedic assistive device 90, each motor controller 84 controlling at least one motor 82 of the orthopedic assistive device 90 in response to a command received from the computer system 92.

Fig. 9 illustrates an embodiment of an orthopedic assistive device 90, which includes a computer system 92. The computer system 92 comprises at least one processor 94, a wireless transmission system 96, and a wireless reception system 98.

The processor 94 is an example of a processing unit within the computer system 92. It is responsible for executing instructions and processing data to perform various functions of the orthopedic assistive device 90. The processor 94 can be a microprocessor, microcontroller, an application-specific integrated circuit ASIC, a field-programmable gate array FPGA, a digital signal processor DSP or any other type of central processing unit CPU. It is typically made of silicon and manufactured using semiconductor fabrication processes. The processor 94 performs computations, controls data flow, and manages tasks.

The wireless transmission system 96 is an example of a communication module within the computer system 92. It is responsible for sending data wirelessly to other devices or systems. The wireless transmission system 96 can utilize at least one wireless communication technology such as Bluetooth, Wi-Fi, an infrared IR transmitter, a Zigbee module, a near-field communication NFC module or cellular networks. It typically consists of a transmitter, an antenna, and associated circuitry. The wireless transmission system 96 enables the orthopedic assistive device 90 to communicate with remote systems or devices, facilitating data exchange and control.

The description of the wireless transmission system 96 applies correspondingly to the wireless reception system 98.

Fig. 10 schematically illustrates an electronic measurement system 86 adapted to measure a myoelectric signal on a human body 70. More specifically, Fig. 10 illustrates an arrangement of sensors S1 - S9 of the electronic measurement system 86.

In the depicted embodiment, the sensors S1 - S9 each comprise an electrode pair. In the prior art, the electrodes of a respective pair are sometimes referred to as the reference electrode and the pickup electrode. The sensors S1 - S9 measure the voltage differences between the electrodes of each of the pairs (i.e., between the reference electrode and the corresponding pickup electrode of the same pair) as the MES, i.e., as the detected MES 12, or as the reference MES described above in the context of the database.

In another embodiment (not shown), the sensors S1 - S9 each comprise an electrode, and a reference electrode (not shown) is provided above/over a position of/on the human body 70, wherein said position of/on the human body 70 is physically distant from the sensors S1 - S9. Preferably, said position of/on the human body 70 is not located directly above/over a muscle. The voltage differences between the reference electrode and each of the electrodes (which may, in such an embodiment, each be referred to as pickup electrodes) are measured as the MES, i.e., as the detected MES 12, or as the reference MES described above in the context of the database.

In yet an alternative embodiment (not shown), the MES is measured as described in the foregoing paragraph, but with the reference electrode located at a position that is not on the human body.

The left panel of Fig. 10 illustrates the anatomy of a human arm and the muscles thereof according to a cross section of the human arm.

The sensors S1 - S9 are arranged around the human arm, such that each one of the sensors S1 - S9 is associated with a muscle of the human arm.

The arrangement of the sensors S1 - S9 is further illustrated in the right panel of Fig. 10.

The method 2 preferably uses the output of each of the sensors S1 - S9. In other words, the detected myoelectric signal 12 comprises a plurality of electric signals (for example, voltage signals) V1_i, V2_i, V3_i, ... V9_i, such that the detected MES 12, for example, takes the following form:

| Time | V1 | V2 | V3 | V4 | V5 | V6 | V7 | V8 | V9 |
|---|---|---|---|---|---|---|---|---|---|
| 0.001 | 1.3 | 1.0 | 0.4 | 1.1 | 0.4 | 0.4 | 0.6 | 0.4 | 0.3 |
| 0.002 | 0.5 | 3.3 | 0.3 | 4.4 | 1.0 | 0.6 | 0.7 | 0.3 | 0.4 |
| 0.003 | 0.3 | 12.5 | 0.1 | 20.4 | 0.4 | 0.7 | 0.6 | 0.3 | 0.4 |
| ... | ... | ... | ... | ... | ... | ... | ... | ... | ... |

In respective embodiments, the reference MES described above in the context of the database are recorded using a similar arrangement of sensors S1 - S9, and thus take a similar form.

As can be seen from the detected MES 12 according to the example above, the muscle contractions of a specific human question induce significant voltages in the electric signals V2 and V4, whereas the electric signals V1, V3, V5 - V9 do not show significant voltages.

The additional information contained in the electric signals V1 - V9, as compared to a dataset consisting of a series of pairs of time stamps t_i and a single series of corresponding voltages V_i, improves the accuracy of the method 2.

Fig. 11 illustrates a client-server system 140 comprising an orthopedic assistive device 90, a client 142, and a remote computer system 144. The orthopedic assistive device 90 comprises a computer system 92.

Fig. 12 shows an example of a computer program 150.

The computer program 150 comprises instructions which, when executed by a computer, cause the computer to carry out the method 2 according to any of the embodiments described above.

According to some embodiments, the computer program 150 comprises instructions which, when executed by a computer system 92, for example the computer system 92 comprised in an orthopedic assistive device 90 as described above, cause said computer system 92 to carry out the method 2 according to any of the embodiments described above.

According to some embodiments, the computer program 150 comprises instructions which, when executed by a client-server system 140, for example, the client-server system 140 as described above, cause said client-server system 140 to carry out the method 2 according to any of the embodiments described above.

Fig. 13 illustrates a non-transitory computer-readable storage medium 160 according to an embodiment. The non-transitory computer-readable storage medium 160 stores the computer program 150 described above.

Fig. 14a, Fig. 14b, and Fig. 14c illustrate the measuring 10 of a myoelectric signal according to an embodiment. In Fig. 14a, Fig. 14b, and Fig. 14c, the horizontal axis corresponds to the time t and the vertical axis corresponds to the voltage V.

First, a continuous myoelectric signal 34 is measured on a human body 70 using the electrodes of an electromyograph, as described above in detail in the context of step 10 of Fig. 1, or as described in the context of Fig. 10. In other words, the myoelectric signal 34 of Fig. 14a is measured continuously on a human body 70, namely in the form of voltage values V_i and corresponding time stamps t_i.

Referring to Fig. 14b, the MES is filtered to remove mains hum (which typically occurs at 50 Hz or 60 Hz and at the respective harmonics) and to remove electrocardiographic signals, which originate from the muscle contractions of the heart muscle. In other words, the dotted line 18 of Fig. 14b is subtracted from the MES 34 of Fig. 14a to arrive at the filtered MES 34' of Fig. 14b.

In Fig. 14b, a beginning 16 and an end 16e of the non-resting phase is detected in the continuous myoelectric signal 34'. The detection is achieved by comparing, during the continuous measurement, the root mean square (RMS) of the last three datapoints to the long-term average (e.g., the average over 1 min) of the RMS of the filtered, continuous MES 34'. In other words, the long-term average (e.g., the average over 1 min) of the RMS is used as a threshold both in the determination of the beginning 16 and of the end 16e of the non-resting phase.

When the continuous measurement of the MES 34, 34' is started, a variable "bool_resting phase" is set to false. When the variable "bool_resting phase" is false, and the RMS of the last three datapoints exceeds the long-term average at a moment in time, the respective moment in time (i.e., the corresponding time stamp t_i) is identified as the beginning 16 of the non-resting phase, and the variable "bool_resting phase" is set to true. When the variable "bool_resting phase" is true, and the RMS of the last three datapoints is below the long-term average at another moment in time, the respective moment in time (i.e., the corresponding time stamp t_i) is identified as the end 16e of the non-resting phase, and the variable "bool_resting phase" is set to false.

The MES 34' (i.e., the pairs of voltage values V_i and corresponding time stamps t_i) from the beginning 16 to the end 16e of the non-resting phase is provided as an MES, i.e., as the detected MES 12 or as the reference MES described above in the context of the database.

As illustrated in Fig. 14c, the detected MES 12 (i.e., the voltage values V_i) from the beginning 16 to the end 16e of the non-resting phase is optionally stretched to a predefined, or pre-selected, time duration 14, respectively. In some embodiments, this means that the detected myoelectric signal 12 is converted into data with a predetermined, or pre-selected, number of data points, respectively, for example using interpolation and/or omission of data points. In respective embodiments, the resulting detected MES 12 (i.e., the pairs of voltage values V_i and corresponding time stamps t_i) is provided as the detected MES 12 or as the reference MES described above in the context of the database.

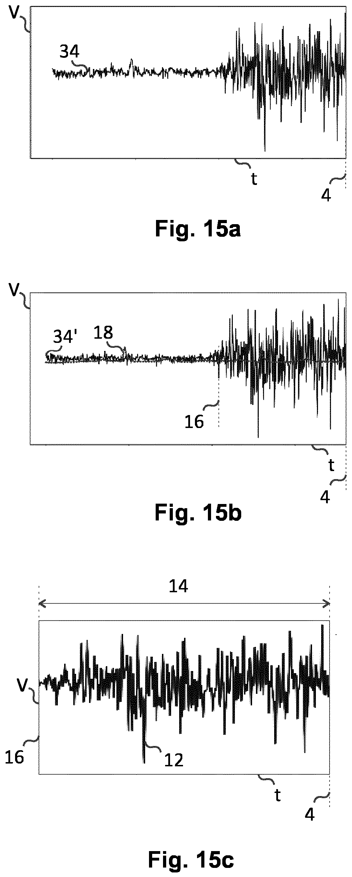

Fig. 15a, Fig. 15b, and Fig. 15c illustrate the detecting 10 of a myoelectric signal 34 according to an alternative embodiment. In Fig. 15a, Fig. 15b, and Fig. 15c, the horizontal axis corresponds to the time t and the vertical axis corresponds to the voltage V.

As illustrated in Fig. 15a, a continuous myoelectric signal 34 is measured on a human body 70. This is performed as described above in the context of Fig. 14a, Fig. 14b, and Fig. 14c.

As illustrated in Fig. 15b, the MES 34 is filtered, corresponding to the subtraction of the curve 18 from the MES 34 and resulting in the filtered, continuous MES 34'. The filtering is performed as described above in the context of Fig. 14a, Fig. 14b, and Fig. 14c.

As illustrated in Fig. 15b, a beginning 16 of a non-resting phase is detected in the filtered, continuous MES 34'. The detection is performed as described above in the context of Fig. 14a, Fig. 14b, and Fig. 14c.

In contrast to the detecting 10 of Fig. 14a, Fig. 14b, and Fig. 14c, the detecting 10 of Fig. 15a, Fig. 15b, and Fig. 15c does not wait for an end 16e of the non-resting phase to occur.

The filtered, continuous MES 34' (i.e., the pairs of voltage values V_i and corresponding time stamps t_i) from the beginning 16 of the non-resting phase until the current moment in time 4 is provided as the detected MES 12.

Optionally, the respective detected MES 12 is stretched to the predefined duration 14 as illustrated in Fig. 15c and described above in the context of Fig. 14c, and the resulting signal is provided as the detected MES 12.

The examples of the present disclosure disclosed herein only constitute specific examples for illustration purposes. The present invention can be implemented in various ways and with many modifications without altering the underlying basic properties. Therefore, the present invention is only defined by the claims as stated below.

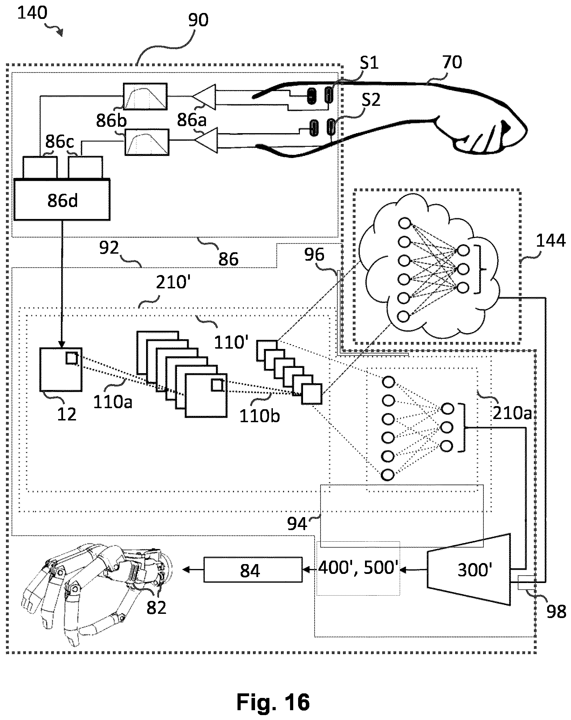

Fig. 16 shows an embodiment of an orthopedic assistive device 90 similar to the one of the foregoing embodiments. Similar elements and method steps are indicated by same reference numerals. For the sake of brevity and to avoid repetition, they will not be described again, but reference is made to the respective description above. In the following, the description of Fig. 16 will focus on modifications over the previous embodiments.

The orthopedic assistive device 90 of the depicted embodiment comprises an electronic measurement system 86 adapted to measure a myoelectric signal on a human body 70, a computer system 92, a motor controller 84, and motors 82.

The electronic measurement system 86 comprises sensors S1, S2 each comprising an electrode pair.

The electronic measurement system 86 further comprises an amplifier system 86a adapted to amplify a signal measured at the sensors S1, S2. The amplifier system includes at least one low-noise, galvanically isolated, high-impedance instrumentation amplifier. Preferably, a respective amplifier is provided for each of the sensors S1, S2.

The electronic measurement system 86 further comprises a filtering system 86b adapted to perform a filtering on the amplified signal from the amplifier system 86a. The signal is filtered to remove mains hum (which typically occurs at 50 Hz or 60 Hz and at the respective harmonics) and to remove electrocardiographic signals, which originate from the muscle contractions of the heart muscle. The filtering is described in more detail above in the context of Fig. 14b and Fig. 15b.

The electronic measurement system 86 further comprises a digital-to-analog converter system 86c adapted to convert the filtered signal from the filtering system 86b into a digital signal.

The electronic measurement system 86 further comprises a digital filtering system 86d adapted to perform a filtering on the digital signal from the digital-to-analog converter system 86c.

The output of the electronic measurement system 86 poses the detected myoelectric signal 12.

The orthopedic assistive device 90, and, more specifically, the computer system 92, comprises a data volume reduction module 110' adapted to perform the process step of deriving 110 data from the detected myoelectric signal 12, as described above in detail.

The data volume reduction module 110' as well as any other module described in the context of Fig. 16 is, in some embodiments, implemented in hardware (e.g., as an ASIC), and, in other embodiments, implemented in software. However, typically, the data volume reduction module 110' as well as any other module described in the context of Fig. 16 is implemented using a combination of hardware and software, namely using the computer system 92 and software installed thereon.

In the depicted embodiment, the process step of deriving 110 data from the detected myoelectric signal 12 comprises process steps 110a, 110b reflecting the application of the first layers of a convolutional neural network. More specifically, at step 110a, at least one convolutional layer of the convolutional neural network is applied. At step 110b, at least one pooling layer of the convolutional neural network is applied. The output of the first layers (i.e., the at least one convolutional layer and the at least one pooling layer) of the convolutional neural network then serves as the data derived from the detected myoelectric signal 12. The inventors have realized that this approach provides an efficient reduction of the data volume associated with the detected myoelectric signal 12.

The data derived from the detected myoelectric signal 12 are sent via the wireless transmission system 96.

The orthopedic assistive device 90, and, more specifically, the computer system 92, comprises a recognition process module 210' adapted to perform the process step of performing 210 a recognition process to determine a second human body gesture 42 corresponding to the detected myoelectric signal 12, as described above in detail.

In the depicted embodiment, the recognition process module 210' comprises the data volume reduction module 110' and a reduced-data processing module 210a.

The reduced-data processing module 210a is adapted to execute the remaining layers of the convolutional neural network on the data derived from the detected myoelectric signal 12 using the data volume reduction module 110', thus completing the recognition process to determine the second human body gesture 42 corresponding to the detected myoelectric signal 12.

The orthopedic assistive device 90, and, more specifically, the computer system 92, comprises a human body gesture determination module 300' adapted to perform process step 300 described above in detail.

For this purpose, the human body gesture determination module 300' is communicatively coupled to the recognition process module 210' to receive the output thereof (i.e., the second human body gesture 42 once available). Further, the human body gesture determination module 300' is communicatively coupled to the wireless reception system 98 of the computer system 92 to check 130 for a response (i.e., to the sending of the data derived from the detected myoelectric signal 12 and sent via the wireless transmission system 96) received using the wireless reception system 98. More specifically, the response being checked for is the first human body gesture 22.

In other words, as soon as the first one of the first human body gesture 22 and the second human body gesture 42 is available at the computer system 92, the respective one of the first one of the first human body gesture 22 and the second human body gesture 42 is determined as the human body gesture 52 associated with the detected myoelectric signal 12 by the human body gesture determination module 300'.

Optionally, the orthopedic assistive device 90, and, more specifically, the computer system 92, comprises a first reliability measure checking module 400' adapted to perform process step 400 described above.

Optionally, the orthopedic assistive device 90, and, more specifically, the computer system 92, comprises a third reliability measure checking module 500' adapted to perform process steps 500, 510, 520 described above.

The computer system 92 passes the output of the human body gesture determination module 300', or, in embodiments with the first reliability measure checking module 400' and/or with the third reliability measure checking module 500', the output of the first reliability measure checking module 400' or of the third reliability measure checking module 500', to the motor controller 84.

The motor controller 84 is adapted to control a movement of the motors 82 of the orthopedic assistive device 90 in response to the human body gesture 52 associated with the detected myoelectric signal 12, as described in detail above.

Fig. 16 further depicts a client-server system 140 comprising the orthopedic assistive device 90 and a remote computer system 144 being remote from the orthopedic assistive device 90.

The remote computer system 144 receives the data derived from the detected myoelectric signal 12 by the data volume reduction module 110' and sent via the wireless transmission system 96.

The remote computer system 144 performs an additional recognition process on the sent, derived data to determine the first human body gesture 22. More specifically, the remote computer system 144 performs the additional recognition process using an additional convolutional neural network. The additional convolutional neural network is larger than the convolutional neural network applied by the reduced-data processing module 210a, i.e., in terms of its parameters or nodes, and/or in terms of the training data it has been trained on.

The remote computer system 144 sends the first human body gesture 22 being the result of the additional recognition process for reception by the wireless reception system 98.

## Claims

1. A computer-implemented method (2) for associating a human body gesture (52) to a detected myoelectric signal (12), the method (2) comprising:
detecting (10) at least a part of a myoelectric signal as the detected myoelectric signal (12);
performing the following first process steps (100), each of said first process steps (100) using a computer system (92) comprised in an orthopedic assistive device (90), said computer system (92) comprising at least one processor (94), a wireless transmission system (96), and a wireless reception system (98):
deriving (110) data from the detected myoelectric signal (12), the derived data representing at least part of an information-content of the detected myoelectric signal (12), a data volume of the derived data being smaller than a data volume of the detected myoelectric signal (12);
sending (120) the derived data using the wireless transmission system (96);
checking (130) for a response received using the wireless reception system (98), said response comprising a first human body gesture (22);
performing (210), before, after, during, or in between the first process steps (100), using the computer system (92) comprised in the orthopedic assistive device (90), a recognition process to determine a second human body gesture (42) corresponding to the detected myoelectric signal (12);
determining (300), by the computer system (92), the one of the first human body gesture (22) and the second human body gesture (42) which is first available at the computer system (92) as the human body gesture (52) associated with the detected myoelectric signal (12).

2. The computer-implemented method (2) of claim 1,
wherein the method (2) further comprises:
checking (400), by the computer system (92), if a first reliability measure is available at the computer system (92), said first reliability measure being associated with the one of the first human body gesture (22) and the second human body gesture (42) which is first available at the computer system (92), wherein said first reliability measure is associated with a likelihood for said one of the first human body gesture (22) and the second human body gesture (42) which is first available at the computer system (92) to correspond to the detected myoelectric signal (12); and
wherein the one of the first human body gesture (22) and the second human body gesture (42) which is first available at the computer system (92) is determined as the human body gesture (52) associated to the detected myoelectric signal (12) if the first reliability measure is available at the computer system (92) and exceeds a first threshold reliability measure value, in particular, only if the first reliability measure is available at the computer system (92) and exceeds the first threshold reliability measure value.

3. The computer-implemented method (2) of claim 1 or 2,
wherein the method (2) further comprises, before, after, during, or in between the first process steps (100):
calculating (220), using the computer system (92), based on the recognition process (20) to determine the second human body gesture (42) corresponding to the detected myoelectric signal (12), a second reliability measure, the second reliability measure being associated with a likelihood for the second human body gesture (42) to correspond to the detected myoelectric signal (12); and
wherein, if the one of the first human body gesture (22) and the second human body gesture (42) which is first available at the computer system (92) is the second human body gesture (42): the second human body gesture (42) is determined as the human body gesture (52) associated to the detected myoelectric signal (12) if the second reliability measure exceeds a second threshold reliability measure value, in particular, only if the second reliability measure exceeds the second threshold reliability measure value.

4. The computer-implemented method (2) of any of the preceding claims,
wherein the recognition process to determine the second human body gesture (42) corresponding to the detected myoelectric signal (12) is performed using a convolutional neural network;
wherein, optionally
the method (2) is the method (2) according to claim 3 and the second reliability measure is calculated using said convolutional neural network; and/or
the data are derived from the detected myoelectric signal (12) using at least a first layer of said convolutional neural network, in particular using at least one convolutional layer of said convolutional neural network and optionally further using at least one pooling layer of said convolutional neural network; and/or
wherein said convolutional neural network is a convolutional neural network on the computer system (92).

5. The computer-implemented method (2) of claim 2, optionally with the features of claim 3 and/or of claim 4,
wherein the method (2) further comprises:
checking (500), by the computer system (92), if a later one of the first human body gesture (22) and the second human body gesture (42) is available at the computer system (92);
checking (510), by the computer system (92), if a third reliability measure associated with the later one of the first human body gesture (22) and the second human body gesture (42) is available at the computer system (92), the third reliability measure being associated with a likelihood for said later one of the first human body gesture (22) and the second human body gesture (42) to correspond to the detected myoelectric signal (12);
if the first reliability measure, the third reliability measure and the later one of the first human body gesture (22) and the second human body gesture (42) are available at the computer system (92):
if the third reliability measure exceeds the first reliability measure: changing (520) the human body gesture (52) associated with the detected myoelectric signal (12) from the one of the first human body gesture (22) and the second human body gesture (42) which is first available at the computer system (92) to the later one of the first human body gesture (22) and the second human body gesture (42), in particular, wherein the human body gesture (52) associated with the detected myoelectric signal (12) is changed from the one of the first human body gesture (22) and the second human body gesture (42) which is first available at the computer system (92) to the later one of the first human body gesture (22) and the second human body gesture (42) only if a time delay between a first moment in time when the detected myoelectric signal (12) is received at the computer system (92) and a second moment in time when the checking (500) if the later one of the first human body gesture (22) and the second human body gesture (42) is available at the computer system (92) has found that the later one of the first human body gesture (22) and the second human body gesture (42) is available at the computer system (92) does not exceed a threshold time delay, in particular, wherein the threshold time delay is associated with a duration of the human body gesture (52) associated with the detected myoelectric signal (12), in particular, wherein the threshold time delay corresponds to half of said duration or to 70% of said duration or to 90% of said duration or to said duration.

6. The computer-implemented method (2) of any of the preceding claims,
wherein, as soon as the first one of the first human body gesture (22) and the second human body gesture (42) is available at the computer system (92), said first one of the first human body gesture (22) and the second human body gesture (42) is determined as the human body gesture (52) associated with the detected myoelectric signal (12); and/or
wherein the checking (130), using the computer system (92), for the response received using the wireless reception system (98), uses a deferred synchronous communication and/or uses a future and/or uses a poll to check for the response received using the wireless reception system (98), in particular, wherein said poll is performed when the computer system (92) is not commanding a motor (82) of the orthopedic assistive device (90) to perform a movement and/or when the computer system (92) does not comprise a pending command to command a motor (82) of the orthopedic assistive device (90), in particular on a memory of the computer system (92); and/or
wherein the orthopedic assistive device (90) is selected from one of the following: a prosthesis, an orthotic device, and an exoskeleton; in particular, wherein the orthopedic assistive device (90) is a prosthesis, in particular, a hand prosthesis; and/or
wherein the recognition process to determine the second human body gesture (42) corresponding to the detected myoelectric signal (12) is performed on the detected myoelectric signal (12) or on the data derived from the detected myoelectric signal (12); and/or
wherein the data are derived from the detected myoelectric signal (12) and the derived data are sent using the wireless transmission system (96) before performing the recognition process to determine the second human body gesture (42) corresponding to the detected myoelectric signal (12).

7. The computer-implemented method (2) of any of the preceding claims, the method (2) further comprising:
receiving (600) the sent, derived data at a remote computer system (144), the remote computer system (144) being remote from the orthopedic assistive device (90);
performing (610), using the remote computer system (144), an additional recognition process on the sent, derived data to determine the first human body gesture (22); and
sending (620), using the remote computer system (144), the determined first human body gesture (22);
in particular, wherein:
in the process step of sending (120) the derived data using the wireless transmission system (96), the derived data are sent to the remote computer system (144) using the wireless transmission system (96); and/or
the process step of checking (130) for the response received using the wireless reception system (98) refers to checking (130), using the wireless reception system (98), for a response received from the remote computer system (144) using the wireless reception system (98); and/or
the determined first human body gesture (22) is sent, using the remote computer system (144), to the computer system (92); and/or
the remote computer system (144) is power-supplied from a power supply providing a larger electrical capacitance than a power supply from which the computer system (92) is power-supplied; and/or
the remote computer system (144) being remote from the orthopedic assistive device (90) comprises an absence of a wired data connection between the computer system (92) and the remote computer system (144).

8. The computer-implemented method (2) of claim 7,
wherein the additional recognition process on the sent, derived data to determine the first human body gesture (22) corresponding to the detected myoelectric signal (12) is performed using an additional convolutional neural network;
wherein, optionally
a fourth reliability measure is calculated, based on the additional recognition process, using said additional convolutional neural network, and wherein the method (2) further comprises sending, using the remote computer, the fourth reliability measure, in particular to the computer system (92); and/or
said additional convolutional neural network is on the remote computer system (144).

9. The computer-implemented method (2) of claim 8, further comprising the features of claim 4,
wherein said data are derived from the detected myoelectric signal (12) using at least a first layer of said convolutional neural network, and
wherein the additional recognition process is performed on said data derived, using the at least first layer of said convolutional neural network, from the detected myoelectric signal (12);
wherein, optionally:
said at least one first layer of said convolutional neural network comprises at least one convolutional layer of said convolutional neural network and optionally further comprises at least one pooling layer of said convolutional neural network; and/or
wherein the data derived from the detected myoelectric signal (12) correspond to the output of said at least one first layer of said convolutional neural network.

10. The computer-implemented method (2) of any of the preceding claims, wherein the method (2) further comprises actuating (54) a plurality of motors (82) according to the human body gesture (52) associated with the detected myoelectric signal (12);
wherein, optionally:
the method (2) further comprises: when the human body gesture (52) associated with the detected myoelectric signal (12) is changed from the one of the first human body gesture (22) and the second human body gesture (42) which is first available at the computer system (92) to the later one of the first human body gesture (22) and the second human body gesture (42), actuating the plurality of motors (82) according to the later one of the first human body (22) gesture and the second human body gesture (42); and/or
said motors (82) are comprised in the orthopedic assistive device (90); and/or
wherein the plurality of motors (82) is actuated to mimic the human body gesture (52) associated with the detected myoelectric signal (12), in particular, to mimic the human body gesture (52) associated with the detected myoelectric signal (12) using said orthopedic assistive device (90); and/or
wherein the method (2) further comprises sending, by the computer system (92), a control command to at least one motor controller (84) adapted to control a movement of said plurality of motors (82), said control command being based on the human body gesture (52) associated with the detected myoelectric signal (12) and being adapted to cause the motor controller (84) to actuate (54) the plurality of motors (82) according to the human body gesture (52) associated with the detected myoelectric signal (12), in particular, wherein the motor controller (84) is comprised in the orthopedic assistive device (90).

11. An orthopedic assistive device (90) comprising a computer system (92), the computer system (92) comprising at least one processor (94), a wireless transmission system (96), and a wireless reception system (98);
wherein the computer system (92) is adapted to:
receive at least a part of a myoelectric signal as a detected myoelectric signal (12);
perform the following first process steps (100):
deriving (no) data from the detected myoelectric signal (12), the derived data representing at least part of an information-content of the detected myoelectric signal (12), a data volume of the derived data being smaller than a data volume of the detected myoelectric signal (12);
sending (120) the derived data using the wireless transmission system (96);
checking (130) for a response received using the wireless reception system (98), said response comprising a first human body gesture (22);
perform (210), before, after, during, or in between the first process steps (100), using the computer system (92) comprised in the orthopedic assistive device (90), a recognition process (20) to determine a second human body gesture (42) corresponding to the detected myoelectric signal (12);
determine the one of the first human body gesture (22) and the second human body gesture (42) which is first available at the computer system (92) as the human body gesture (52) associated with the detected myoelectric signal (12).

12. The orthopedic assistive device (90) according to claim 11, further comprising:
an electronic measurement system (86) adapted to measure a myoelectric signal on a human body (70), and to provide said myoelectric signal to the computer system (92) for being received as the at least part of the myoelectric signal; and/or
a plurality of motors (82), wherein the orthopedic assistive device (90) is adapted to actuate the plurality of motors (82) according to the human body gesture (52) associated with the detected myoelectric signal (12), in particular, to mimic the human body gesture (52) associated with the detected myoelectric signal (12).

13. The orthopedic assistive device (90) according to claim 11 or 12,
wherein the orthopedic assistive device (90) is one of the following: a prosthesis, an orthotic device, and an exoskeleton; in particular, wherein the orthopedic assistive device (90) is a prosthesis, such as a hand prosthesis.

14. A client-server system (140) comprising the orthopedic assistive device (90) according to any of claims 11 to 13, a client (142) of the client-server system (140) being provided or being provided at least in part by the computer system (92) of the orthopedic assistive device (90), the client-server system (140) further comprising a remote computer system (144) being remote from the orthopedic assistive device (90), the remote computer system (144) adapted to perform the following process steps:
receiving the sent, derived data;
performing an additional recognition process on the sent, derived data to determine the first human body gesture (22); and
sending the determined first human body gesture (22).

15. A computer program (150) comprising instructions which, when the computer program (150) is executed by a computer, cause the computer to carry out the method (2) according to any of claims 1 to 10,
in particular, wherein the computer program (150) comprises instructions which:
when the computer program (150) is executed by a computer system (92), cause the computer system (92) to carry out the method (2) according to any of claims 1 to 10, or
when the computer program (150) is executed by a client-server system (140), cause the client-server system (140) system to carry out the method (2) according to any of claims 7 to 10.
